# EUROPEAN PATENT SPECIFICATION

(11) **EP 2 389 197 B1**
(45) Date of publication and mention of the grant of the patent: **18.06.2014**
(21) Application number: 10701206.4
(22) Date of filing: 19.01.2010
(51) Int. Cl.: A61K 45/06, A61K 31/519, A61K 31/44, A61P 29/00

(54) **COMBINATIONS COMPRISING METHOTREXATE AND DHODH INHIBITORS**
KOMBINATIONEN, DIE METHOTREXAT UND DHODH-INHIBITOREN UMFASSEN
COMBINAISONS COMPORTANT DES INHIBITEURS DHODH ET DE LA MÉTHOTREXATE

(30) Priority: 21.01.2009 EP 09382006
(43) Date of publication of application: 30.11.2011
(73) Proprietor: Almirall S.A., 08022 Barcelona (ES)
(72) Inventor: GODESSART MARINA, Nuria, 08980 Sant Feliu de Llobregat (Barcelona) (ES); PIZCUETA LALANZA, Maria, Pilar, 08980 Sant Feliu de Llobregat (Barcelona) (ES)
(74) Representative: Srinivasan, Ravi Chandran
(86) International application number: PCT/EP2010/000270
(87) International publication number: WO 2010/083975

(56) References cited:
- WO-A-2009/021696
- KREMER J M: "Methotrexate and leflunomide: Biochemical basis for combination therapy in the treatment of rheumatoid arthritis" SEMINARS IN ARTHRITIS AND RHEUMATISM, XX, XX, vol. 29, no. 1, 1 August 1999 (1999-08-01) , pages 14-26, XP004680855 ISSN: 0049-0172
- LEBAN J ET AL: "Biphenyl-4-ylcarbamoyl thiophene carboxylic acids as potent DHODH inhibitors" BIOORGANIC & MEDICINAL CHEMISTRY LETTERS, PERGAMON, ELSEVIER SCIENCE, GB, vol. 16, no. 2, 15 January 2006 (2006-01-15), pages 267-270, XP025106511 ISSN: 0960-894X [retrieved on 2006-01-15]
- SES RESEARCH: "Company Report: 4SC" INTERNET CITATION, [Online] XP002440315 Retrieved from the Internet: URL:http://www.4sc.de/en/investors/media/4 SC-Analystenreport-eng.pdf> [retrieved on 2007-01-01]

## Description

The present invention relates to new combinations of methotrexate with DHODH inhibitors. These combinations are useful in the treatment, prevention or suppression of diseases and disorders known to be susceptible to improvement with methotrexate and / or by inhibition of dihydroorotate dehydrogenase, such as autoimmune diseases, immune and inflammatory diseases, destructive bone disorders, malignant neoplastic diseases, angiogenic-related disorders, viral diseases, and infectious diseases.

### BACKGROUND OF THE INVENTION

Methotrexate (MTX) is an antimetabolite and immunomodulator that affects many intracellular pathways of purine metabolism. It is effective in reducing the signs and symptoms of rheumatoid arthritis (RA), as well as in slowing or halting radiographic damage. Due to its efficacy, ease of administration and relatively low cost, MTX has become the first-line oral therapy in most patients with RA. In those patients who have an incomplete response to MTX, another DMARD (disease modifying anti-rheumatic drug) is added on top of it. Thus, combination therapy with MTX is more and more frequent in the clinical practice.

Leflunomide is an example of such a DMARD. It was approved in September 1998 for use in RA. It has been shown to reduce the signs and symptoms of the disease, to inhibit structural damage (evidenced by X-ray erosions and joint space narrowing) and to improve physical function. Teriflunomide is the active metabolite of Leflunomide.

Methotrexate is thought to act primarily on purine pathways of cellular metabolism, whereas Leflunomide affects pyrimidine pathways. Given the diverse intracellular pathways affected by both drugs, the combination of Leflunomide and methotrexate has the potential for biochemical synergy. In fact, it has been reported that the combination of both agents led to considerable clinical improvement (see for example, Weinblatt ME et al. "Pharmacokinetics, safety, and efficacy of combination treatment with methotrexate and leflunomide in patients with active rheumatoid arthritis". Arthritis Rheum 1999; 42 (7): 1322-8 and Kremer JM et al. "Concomitant Leflunomide therapy in patients with active rheumatoid arthritis despite stable doses of methotrexate" Ann. Intern. Med., 2002; 137, 72-733).

Unfortunately, both methotrexate and leflunomide have serious adverse effects, in particular hepatotoxicity. Methotrexate may cause fatal liver damage such as fibrosis and cirrhosis after prolonged use. Liver enzyme increases are frequently seen during treatment with methotrexate. Hence, regular and careful monitoring of patients taking MTX is essential, particularly when MTX is combined with other DMARDs.

The most common reported adverse events of Leflunomide include diarrhoea, dyspepsia, rash, hair loss, hypertension and elevated hepatic enzymes. The hepatotoxicity potential is of special relevance and regular laboratory tests, including blood tests of liver function, must be performed for all patients taking this medication. Leflunomide is not recommended for use in patients with evidence of hepatitis B or C infection or significant hepatic impairment.

Clinical trials have reported that the number of patients experiencing an increase in liver markers (measured as transaminase levels) is notably higher in the group of Leflunomide plus MTX than in the group of MTX alone. The product information for Leflunomide warns against combination with methotrexate on the basis that such combination therapy can lead to additive or even synergistic hepatotoxicity.

The mechanism responsible for the hepatotoxicity of leflunomide, and in particular of its active metabolite, teriflunomide, is unknown, but it has been attributed to its activity as inhibitor of dihydroorotate dehydrogenase (DHODH). Liver toxicity has thus been identified as an adverse effect directly derived from the mechanism of action of DHODH-inhibitors, which has hampered the development of this class of compounds.

### DESCRIPTION OF THE INVENTION

It has now been found that, contrary to general belief, inhibition of DHODH is not responsible for the liver damage produced by leflunomide and that certain non-hepatotoxic DHODH inhibitors are particularly suitable for combination with methotrexate.

It is known that inhibition of DHODH produces immunosuppressant and antiproliferative effects. DHODH inhibitors can therefore be used as immunosuppressants and as antiproliferatives in the treatment of autoimmune, inflammatory and proliferative diseases, like RA.

The present invention is based on the surprising finding that the inhibition of DHODH is not linked to hepatotoxicity and, consequently, DHODH inhibitors devoid of hepatotoxic potential represent an important contribution to the treatment of these diseases, due to their advantageous combinability with MTX, the most commonly used first-line drug in RA treatment.

We have developed an *in vivo* model of hepatotoxicity assessment in mice, in which test compounds are administered by intraperitoneal route to maximise liver exposure. In this model, Teriflunomide, the active metabolite of Leflunomide, has shown a drastic increase in the levels of transaminases and bilirrubin in plasma, whereas DHODH inhibitors do not show an increase in any of the plasma liver markers in the same model, while maintaining their efficacy in arthritis.

Thus, the present invention is directed to a combination for simultaneous, separate or sequential use in the treatment of a pathological condition or disease susceptible to amelioration by inhibition of dihydroorotate dehydrogenase which is selected from rheumatoid arthritis, psoriatic arthritis, ankylosing spondilytis, multiple sclerosis, Wegener's granulomatosis, systemic lupus erythematosus, psoriasis and sarcoidosis, which comprises (a) methotrexate and (b) a non-hepatotoxic DHODH inhibitor of formula (I): 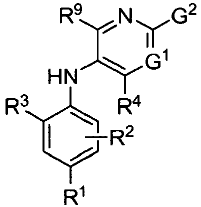 wherein:
R¹ is selected from the group consisting of hydrogen atoms, halogen atoms, C₁₋₄ alkyl, C₃₋₄ cycloalkyl, -CF₃ and -OCF₃,
R² is selected from the group consisting of hydrogen atoms, halogen atoms and C₁₋₄ alkyl groups,
R³ is selected from the group consisting of -COOR⁵, -CONHR⁵, tetrazolyl, -SO₂NHR⁵ and -CONHSO₂R⁵ groups, wherein R⁵ is selected from the group consisting of a hydrogen atom and linear or branched C₁₋₄ alkyl groups,
R⁴ is selected from the group consisting of a hydrogen atom and a C₁₋₄ alkyl group,
R⁹ is selected from the group consisting of a hydrogen atom and a phenyl group,
G¹ represents a group selected from N and CR⁶ wherein R⁶ is selected from the group consisting of hydrogen atoms, halogen atoms, C₁₋₄ alkyl, C₃₋₄ cycloalkyl, C₁₋₄ alkoxy, -CF₃, -OCF₃, monocyclic N-containing C₅₋₇ heteroaryl, monocyclic N-containing C₃₋₇ heterocyclyl groups and C₆₋₁₀ aryl groups which C₆₋₁₀ aryl groups are optionally substituted with one or more substituents selected from halogen atoms and C₁₋₄ alkyl groups,
G² represents a group selected from:
   - a hydrogen atom, a hydroxy group, a halogen atom, a C₃₋₄ cycloalkyl group, a C₁₄ alkoxy group and -NR^{a}R^{b}, wherein
      R^{a} represents a C₁₋₄ alkyl group and R^{b} is selected from a group consisting of C₁₋₄ alkyl group and C₁₋₄ alkoxy-C₁₋₄ alkyl group, or
      R^{a} and R^{b} together with the nitrogen atom to which they are attached form a saturated 6 to 8 membered heterocyclic ring optionally containing one oxygen atom as an additional heteroatom,
   - a monocyclic or bicyclic 5 to 10 membered heteroaromatic ring containing one or more nitrogen atoms which is optionally substituted by one or more substituents selected from halogen atoms, C₁₋₄ alkyl, C₁₋₄ alkoxy, C₃₋₄ cycloalkyl, C₃₋₄ cycloalkoxy, -CF₃, -OCF₃, and -CONR⁷R⁸, wherein R⁷ and R⁸ are independently selected from hydrogen atom, linear or branched C₁₋₄ alkyl groups, C₃₋₇ cycloalkyl groups, or R⁷ and R⁸ together with the nitrogen atom to which they are attached form a group of formula 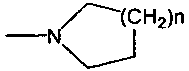 wherein n is an integer from 0 to 3,
      and
   - a phenyl group which is optionally substituted by one or more substituents selected from halogen atoms, C₁₋₄ alkyl, hydroxyl, C₁₋₄ alkoxy, C₃₋₄cycloalkyl, C₃₋₄ cycloalkoxy, cyano, -CF₃, -OCF₃, -CONR⁷R⁸, oxadiazolyl, triazolyl, pyrazolyl and imidazolyl groups, which oxadiazolyl, triazolyl, pyrazolyl and imidazolyl groups are optionally substituted by C₁₋₄ alkyl or C₃₋₇ cycloalkyl groups and wherein R⁷ and R³ are independently selected from hydrogen atom, linear or branched C₁₋₄ alkyl groups, C₃₋₇ cycloalkyl groups, or R⁷ and R⁸ together with the nitrogen atom to which they are attached form a group of formula 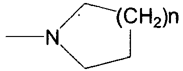
wherein n is an integer from 0 to 3
or, when G' represents CR⁶, G² together with R⁶ forms a non-aromatic C₅₋₁₀ carbocyclic group or a C₆₋₁₀ aryl group,
and the pharmaceutically acceptable salts and N-oxides thereof,
wherein
(i) the methotrexate (a) is for administration at a dosage regime which involves administration of 0.015 to 3 mg/kg/week of methotrexate and the DHODH inhibitor (b) is for administration at a dosage regime which involves administration of 0.03 to 30 mg/kg/day of DHODH inhibitor; or
(ii) the combination is for use in treating a human or animal patient suffering from hepatic impairment, liver fibrosis, hepatitis, cirrhosis or liver cancer.

The present invention also provides a DHODH inhibitor as defined above for use in treating a human or animal patient suffering from or susceptible to a pathological condition or disease susceptible to amelioration by inhibition of dihydroorotate dehydrogenase as defined above, wherein the human or animal patient is suffering from hepatic impairment, liver fibrosis, cirrhosis or liver cancer.

As used herein the term alkyl embraces optionally substituted, linear or branched hydrocarbon radicals having 1 to 4 carbon atoms. Preferred substituents on the alkyl groups are halogen atoms and hydroxy groups.

Examples include methyl, ethyl, *n*-propyl, *i*-propyl, *n*-butyl, *sec*-butyl and *tert*-butyl radicals.

As used herein the term alkoxy embraces optionally substituted, linear or branched oxygen containing radicals each having 1 to 4 carbon atoms. Preferred substituents on the alkoxy groups are halogen atoms and hydroxy groups.

Examples include methoxy, ethoxy, *n*-propoxy, *i*-propoxy, *n*-butoxy, *sec*-butoxy and *tert-*butoxy radicals.

As used herein, the term cycloalkyl embraces optionally substituted saturated carbocyclic radicals and, unless otherwise specified, a cycloalkyl radical typically has from 3 to 7 carbon atoms, preferably from 3 to 4 carbon atoms.

Examples include cyclopropyl, cyclobutyl, cyclopentyl, cyclohexyl and cycloheptyl. When a cycloalkyl radical carries 2 or more substituents, the substituents may be the same or different. Preferred substiuents on the cycloalkyl groups are halogen atoms and hydroxy groups.

As used herein, the term cycloalkoxy embraces saturated oxy-containing carbocyclic radicals and, unless otherwise specified, a cycloalkoxy radical typically has from 3 to 8 carbon atoms, preferably from 3 to 4 carbon atoms.

Examples include cyclopropyloxy, cyclobutyloxy, cyclopentyloxy, cyclohexyloxy and cycloheptyloxy. When a cycloalkoxy radical carries 2 or more substituents, the substituents may be the same or different. Preferred substiuents on the cycloalkoxy groups are halogen atoms and hydroxy groups.

As used herein, the term aryl radical embraces typically optionally substituent C₆-C₁₀ monocyclic or polycyclic aryl radical such as phenyl, naphthyl, anthranyl and phenanthryl. Phenyl is preferred.

A said optionally substituted aryl radical is typically unsubstituted or substituted with 1, 2 or 3 substituents which may be the same or different. The substituents are preferably selected from halogen atoms, preferably fluorine atoms, hydroxy groups, alkoxycarbonyl groups in which the alkyl moiety has from 1 to 4 carbon atoms, hydroxycarbonyl groups, carbamoyl groups, nitro groups, cyano groups, C₁-C₄ alkyl groups, C₁-C₄ alkoxy groups and C₁-C₄ hydroxyalkyl groups. When an aryl radical carries 2 or more substituents, the substituents may be the same or different. Unless otherwise specified, the substituents on an aryl group are typically themselves unsubstituted.

As used herein, the terms heteroaryl and heteroaromatic ring are used interchangeably and are typically 5- to 14- membered ring systems, preferably 5- to 10- membered ring systems, comprising at least one heteroaromatic ring and containing at least one heteroatom selected from O, S and N. A heteroaryl radical may be a single ring (monocyclic) or two or more fused rings (polycyclic) wherein at least one ring contains a heteroatom.

As used herein, the term heterocyclyl radical is typically a non-aromatic, saturated or unsaturated C₃-C₁₀ carbocyclic ring system, such as a 5, 6 or 7 membered radical, in which one or more, for example 1, 2, 3 or 4 of the carbon atoms preferably 1 or 2 of the carbon atoms are replaced by a heteroatom selected from N, O and S. Saturated heterocyclyl radicals are preferred.

As used herein, the term halogen atom refers typically to chlorine, fluorine, bromine and iodine atoms, preferably fluorine, chlorine and bromine atoms. The term halo when used as a prefix has the same meaning.

As used herein, some of the atoms, radicals, moieties, chains or cycles present in the general structures of the invention are "optionally substituted". This means that these atoms, radicals, moieties, chains or cycles can be either unsubstituted or substituted in any position by one or more, for example 1, 2, 3 or 4, substituents, whereby the hydrogen atoms bound to the unsubstituted atoms, radicals, moieties, chains or cycles are replaced by chemically acceptable atoms, radicals, moieties, chains or cycles. When two or more substituents are present, each substituent may be the same or different.

As used herein, the term pharmaceutically acceptable salt embraces salts with a pharmaceutically acceptable acid or base. Pharmaceutically acceptable acids include both inorganic acids, for example hydrochloric, sulphuric, phosphoric, diphosphoric, hydrobromic, hydroiodic and nitric acid and organic acids, for example citric, fumaric, maleic, malic, mandelic, ascorbic, oxalic, succinic, tartaric, benzoic, acetic, methanesulphonic, ethanesulphonic, benzenesulphonic, cyclohexylsulfamic (cyclamic) or p-toluenesulphonic acid. Pharmaceutically acceptable bases include alkali metal (e.g. sodium or potassium) and alkali earth metal (e.g. calcium or magnesium) hydroxides and organic bases, for example alkyl amines, arylalkyl amines and heterocyclic amines.

Other preferred salts according to the invention are quaternary ammonium compounds wherein an equivalent of an anion (X⁻) is associated with the positive charge on the N atom. X⁻ may be an anion of various mineral acids such as, for example, chloride, bromide, iodide, sulphate, nitrate, phosphate, or an anion of an organic acid such as, for example, acetate, maleate, fumarate, citrate, oxalate, succinate, tartrate, malate, mandelate, trifluoroacetate, methanesulphonate and *p*-toluenesulphonate. X⁻ is preferably an anion selected from chloride, bromide, iodide, sulphate, nitrate, acetate, maleate, oxalate, succinate or trifluoroacetate. More preferably X⁻ is chloride, bromide, trifluoroacetate or methanesulphonate.

In the particular case where R³ is a COOH group, it is advantageous to have salts derived from the corresponding carboxylic acid by replacement of the hydrogen atom of the carboxylic group with a cation derived from a pharmaceutically acceptable base as described above.

As used herein, an N-oxide is formed from the tertiary basic amines or imines present in the molecule, using a convenient oxidising agent.

Typically, R¹ is selected from the group consisting of hydrogen atoms, fluorine atoms, chlorine atoms, bromine atoms, C₁₋₄ alkyl, C₃₋₄ cycloalkyl and -CF₃ groups.

Typically R² is selected from the group consisting of hydrogen atoms, halogen atoms and methyl groups.

Typically, G¹ is selected from the group consisting of nitrogen atoms, CCl, CF, CH, C(CH₃), C(cyclopropyl), C(phenyl) and C(CF₃) groups.

Typically G² represents a group selected from:
- a hydrogen atom, a halogen atom, a C₃₋₄ cycloalkyl group, a C₁₋₂ alkoxy group and -NR^{a}R^{b}, wherein
   R^{a} represents a C₁₋₂ alkyl group and R^{b} is selected from the group consisting of C_{1.2} alkyl groups and C₁₋₂ alkoxy-C₁₋₂ alkyl groups, or
   R^{a} and R^{b} together with the nitrogen atom to which they are attached form a saturated 6 or 7 membered heterocyclic ring optionally containing one oxygen atom as an additional heteroatom,
- a monocyclic or bicyclic 5 to 10 membered heteroaromatic ring containing one or two nitrogen atoms which is optionally substituted by one or more substituents selected from halogen atoms and C₁₋₄ alkyl groups,
   and
- a phenyl group which is optionally substituted by one, two or three substituents selected from halogen atoms, C₁₋₄ alkyl, hydroxyl, C₁₋₄ alkoxy, C₃₋₄cycloalkyl, C₃₋₄ cycloalkoxy, cyano, -CF₃, -OCF₃, -CONR⁷R⁸ and oxadiazolyl groups, which oxadiazolyl groups are optionally substituted by a C₁₋₄ alkyl or a C₃₋₇ cycloalkyl group and wherein R⁷ and R⁸ are independently selected from hydrogen atoms, linear or branched C₁₋₄ alkyl groups, C₃₋₄ cycloalkyl groups, or R⁷ and R⁸ together with the nitrogen atom to which they are attached form a group of formula 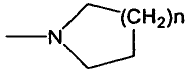
wherein n is 1 or 2,
or, when G' represents CR⁶, G² together with R⁶ forms a non-aromatic C₆ carbocyclic group or a phenyl group.

More typically G² represents a group selected from:
- a hydrogen atom, a fluorine atom, a cyclopropyl group, a methoxy group, -NMeEt, -NEt₂, -N(Me)-(CH₂)₂-O-CH₃, 6-morpholinyl, azepan-1-yl and piperidin-1-yl,
- a pyridinyl, pyrimidinyl, quinolinyl or pyrazinyl ring optionally substituted with one or two substituents selected from Me and F
   and
- a phenyl group which is optionally substituted by one, two or three substituents selected from fluorine, chlorine, methyl, hydroxyl, methoxy, ethoxy, isopropyloxy, cyclopropyl, cyclopropyloxy, cyano, -CF₃, -OCF₃ ,oxadiazolyl and -CONR⁷R⁸ groups, which oxadiazolyl groups are optionally substituted by a methyl group and wherein R⁷ and R⁸ are independently selected from hydrogen atom, methyl group, isopropyl group, cyclopropyl group, or R⁷ and R⁸ together with the nitrogen atom to which they are attached form a group of formula 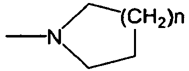
wherein n is 1,
or, when G' represents CR⁶, G² together with R⁶ forms a non-aromatic C₆ carbocyclic group or a phenyl group.

Even more typically, G² represents a group selected from methoxy group, cyclopropyl group and optionally substituted phenyl, pyridyl, quinolynyl, pyrimidinyl and pyrazinyl groups.

In one embodiment of the present invention, R⁹ represents a hydrogen atom, and G² represents a group selected from:
- a monocyclic or bicyclic 5 to 10 membered heteroaromatic ring containing a nitrogen atom which is optionally substituted by one or more substituents selected from halogen atoms, C₁₋₄ alkyl, C₁₋₄ alkoxy, C₃₋₄ cycloalkyl, C₃₋₄ cycloalkoxy, -CF₃, -OCF₃, and -CONR⁷R⁸, wherein R⁷ and R⁸ are independently selected from hydrogen atom, lineal or branched C₁₋₄ alkyl group, C₃₋₇ cycloalkyl group, or R⁷ and R⁸ together with the nitrogen atom to which they are attached form a group of formula 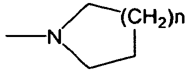 wherein n is an integer from 0 to 3,
   and
- a phenyl group which is optionally substituted by one or more substituents selected from halogen atoms, C₁₋₄ alkyl, C₁₋₄ alkoxy, C₃₋₄cycloalkyl, C₃₋₄ cycloalkoxy, -CF₃, -OCF₃, -CONR⁷R⁸, oxadiazolyl, triazolyl, pyrazolyl and imidazolyl groups, wherein the oxadiazolyl, triazolyl, pyrazolyl and imidazolyl groups are optionally substituted by C₁₋₄ alkyl or C₃₋₇ cycloalkyl group and wherein R⁷ and R⁸ are independently selected from hydrogen atom, lineal or branched C₁₋₄ alkyl group, a C₃₋₇ cycloalkyl group, or R⁷ and R⁸ together with the nitrogen atom to which they are attached form a group of formula 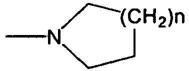
wherein n is an integer from 0 to 3
and the pharmaceutically acceptable salts and N-oxides thereof.

Typically, R' is selected from the group consisting of C₁₋₄ alkyl, C₃₋₄ cycloalkyl and -CF₃, preferably methyl and cyclopropyl group, more preferably a cyclopropyl group.

Typically, R² is selected from a hydrogen or halogen atom, preferably a hydrogen atom.

Typically, R³ is selected from COOR⁵, -CONHR⁵ and tetrazolyl group; preferably R³ is a COOH group.

Typically, R⁴ represents a hydrogen atom or a methyl group, preferably a hydrogen atom.

Typically, R⁹ represents a hydrogen atom.

Typically, G' represents a group selected from N, CH, C(CH₃), C(cyclopropyl), C(phenyl) or C(CF₃) groups.

Typically, G² is selected from the group consisting of a methoxy group, a cyclopropyl group and optionally substituted phenyl, pyridyl, quinolynyl, pyrimidinyl and pyrazinyl groups, more preferably, G² is selected from the group consisting of optionally substituted phenyl, pyridyl, quinolynyl, pyrimidinyl and pyrazinyl groups, most preferably selected from optionally substituted phenyl, 4-pyridyl, 5-quinolynyl and 2-pyrazinyl groups.

In yet another embodiment of the present invention, R' is selected from a methyl or cyclopropyl group, R² represents a hydrogen atom, R³ is a COOH group, R⁴ represents a hydrogen atom or a methyl group, G¹ is selected from N, CH, C(CH₃), C(cyclopropyl), C(phenyl) and C(CF₃) groups, and G² represents a group selected from the group consisting of optionally substituted phenyl, 4-pyridyl, 5-quinolynyl and 2-pyrazinyl groups, more preferably R⁹ represent a hydrogen group.

In yet another embodiment of the present invention, R' is selected from a methyl or cyclopropyl group, R² represents a hydrogen atom, R³ is a COOH group, R⁴ represents a hydrogen atom, G¹ is selected from nitrogen atoms and CH, C(CH₃) and C(CF₃) groups and G² represents a phenyl group optionally substituted with one or two substituents selected from chloro, fluoro, methoxy, ethoxy, isopropoxy, trifluoromethoxy and -CONR⁷R⁸, wherein R⁷ is hydrogen and R⁸ is cyclopropyl or R⁷ and R⁸ together with the nitrogen atom to which they are attached form a group of formula 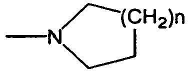 wherein n is 1.

Preferably, the DHODH inhibitor is one of the following list:
1. 5-cyclopropyl-2-(2-phenylpyrimidin-5-ylamino)benzoic acid;
2. 2-(6-Cyclopropyl-5-methylpyridin-3-ylamino)-5-methylbenzoic acid;
3. 5-(2-Carboxy-4-cyclopropylphenylamino)-3-methyl-2-phenylpyridine 1-oxide;
4. 5-Methyl-2-(6-(3-(trifluoromethyl)phenyl)pyridin-3-ylamino)benzoic acid;
5. 5-cyclopropyl-2-(6-hydroxy-5-phenylpyridin-3-ylamino)benzoic acid;
6. 5-cyclopropyl-2-(2-(2,6-difluoro-4-hydroxyphenyl)pyrimidin-5-ylamino)benzoic acid;
7. 5-Cyclopropyl-2-(6-methoxy-5-phenylpyridin-3-ylamino) benzoic acid;
8. 2-(5-Fluoro-6-phenylpyridin-3-ylamino)-5-methylbenzoic acid;
9. 2-(6-(Ethyl(methyl)amino)-5-methylpyridin-3-ylamino)-5-methylbenzoic acid;
10. 5-Cyclopropyl-2-(3'-fluoro-2,4'-bipyridin-5-ylamino)benzoic acid;
11. 2-(6-(Diethylamino)-5-methylpyridin-3-ylamino)-5-methylbenzoic acid;
12. 2-(6-((2-Methoxyethyl)(methyl)amino)-5-methylpyridin-3-ylamino)-5-methylbenzoic acid;
13. 2-(5-Chloro-6-phenylpyridin-3-ylamino)-5-methylbenzoic acid;
14. 5-Cyclopropyl-2-(2-(2-cyclopropylphenyl)pyrimidin-5-ylamino)benzoic acid;
15. 5-cyclopropyl-2-(5-phenylpyridin-3-ylamino) benzoic acid;
16. 5-methyl-2-(quinolin-3-ylamino)benzoic acid;
17. 5-methyl-2-(5,6,7,8-tetrahydroquinolin-3-ylamino)benzoic acid;
18. 2-(5-Chloro-2-phenylpyridin-3-ylamino)-5-methylbenzoic acid;
19. 5-Cyclopropyl-2-(5,6-diphenylpyridin-3-ylamino)benzoic acid;
20. 5-cyclopropyl-2-(2-(2,6-difluorophenyl)pyrimidin-5-ylamino)benzoic acid;
21. 5-Cyclopropyl-2-(5-methylpyridin-3-ylamino) benzoic acid;
22. 2-(2-(3-Cyclopropoxyphenyl)pyrimidin-5-ylamino)-5-cyclopropylbenzoic acid;
23. 5-Methyl-2-(6-morpholinopyridin-3-ylamino)benzoic acid;
24. 5-Methyl-2-(5-methyl-6-morpholinopyridin-3-ylamino)benzoic acid;
25. 5-cyclopropyl-2-(6-cyclopropyl-5-phenylpyridin-3-ylamino) benzoic acid;
26. 2-(6-(2-Cyclopropylphenyl)-5-methylpyridin-3-ylamino)-5-methylbenzoic acid;
27. 2-(6-(2-Cyanophenyl)-5-methylpyridin-3-ylamino)-5-methylbenzoic acid;
28. 2-(2-(3-Chlorophenyl)pyrimidin-5-ylamino)-5-cyclopropylbenzoic acid;
29. 5-Methyl-2-(6-phenyl-5-(trifluoromethyl)pyridin-3-ylamino)benzoic acid;
30. 5-Methyl-2-(5-methyl-6-(piperidin-1-yl)pyridin-3-ylamino)benzoic acid;
31. 2-(6-(Azepan-1-yl)-5-methylpyridin-3-ylamino)-5-methylbenzoic acid;
32. 2-(6-(3-Methoxyphenyl)-5-phenylpyridin-3-ylamino)-5-methylbenzoic acid;
33. 2-(2,3'-bipyridin-5-ylamino)-5-cyclopropylbenzoic acid;
34. 2-(3'-chloro-2,4'-bipyridin-5-ylamino)-5-methylbenzoic acid;
35. 5-Methyl-2-(3-methyl-2,2'-bipyridin-5-ylamino)benzoic acid;
36. 2-(5,6-Difluoropyridin-3-ylamino)-5-methylbenzoic acid;
37. 2-(6-(3-Methoxyphenyl)pyridin-3-ylamino)benzoic acid;
38. 2-(6-(3-Ethoxyphenyl)pyridin-3-ylamino)benzoic acid;
39. 2-(6-(3-Ethoxyphenyl)pyridin-3-ylamino)-5-fluorobenzoic acid;
40. 2-(6-(3-Ethoxyphenyl)-5-methylpyridin-3-ylamino)benzoic acid;
41. 2-(6-(3-Ethoxyphenyl)pyridin-3-ylamino)-5-methylbenzoic acid;
42. 2-(6-(3-Ethoxyphenyl)-5-methylpyridin-3-ylamino)-5-methylbenzoic acid;
43. 2-(6-(3-Ethoxy-2-fluorophenyl)pyridin-3-ylamino)benzoic acid;
44. 2-(6-(3-Ethoxyphenyl)-4-methylpyridin-3-ylamino)-5-methylbenzoic acid;
45. 2-(6-(3-Ethoxyphenyl)-4-methylpyridin-3-ylamino)benzoic acid;
46. 5-Bromo-2-(6-(3-ethoxyphenyl)pyridin-3-ylamino)benzoic acid;
47. 5-Chloro-2-(6-(3-ethoxyphenyl)pyridin-3-ylamino)benzoic acid;
48. 2-(6-(5-Ethoxy-2-fluorophenyl)pyridin-3-ylamino)benzoic acid;
49. 2-(6-(3-Ethoxyphenyl)-5-methylpyridin-3-ylamino)-5-(trifluoromethyl)benzoic acid;
50. 2-(6-(3-Methoxyphenyl)-5-methylpyridin-3-ylamino)-5-(trifluoromethyl)benzoic acid;
51. 2-(6-(3-Methoxyphenyl)-5-methylpyridin-3-ylamino)-5-methylbenzoic acid;
52. 2-(6-(3-Methoxyphenyl)-5-methylpyridin-3-ylamino)-6-methylbenzoic acid;
53. 5-Fluoro-2-(6-(3-methoxyphenyl)-5-methylpyridin-3-ylamino)benzoic acid;
54. 2-(6-(5-Ethoxy-2-fluorophenyl)pyridin-3-ylamino)-5-methylbenzoic acid;
55. 2-(6-(2-Fluoro-5-methoxyphenyl)-5-methylpyridin-3-ylamino)-5-methylbenzoic acid;
56. Ethyl 2-(6-(2-fluoro-5-methoxyphenyl)-5-methylpyridin-3-ylamino)-5-methylbenzoate;
57. 2-(6-(2-Fluorophenyl)pyridin-3-ylamino)-5-methylbenzoic acid;
58. 2-(6-(3-Methoxyphenyl)-5-phenylpyridin-3-ylamino)-5-methylbenzoic acid;
59. Ethyl 2-(6-(3-methoxyphenyl)-5-phenylpyridin-3-ylamino)-5-methylbenzoate;
60. 5-Methyl-2-(5-methyl-6-phenylpyridin-3-ylamino)benzoic acid;
61. Ethyl 5-methyl-2-(5-methyl-6-phenylpyridin-3-ylamino)benzoate;
62. 5-Methyl-2-(5-methyl-6-(3-(trifluoromethoxy)phenyl)pyridin-3-ylamino)benzoic acid;
63. Ethyl 5-methyl-2-(5-methyl-6-(3-(trifluoromethoxy)phenyl)pyridin-3-ylamino)benzoate;
64. 2-(5-Cyclopropyl-6-(3-methoxyphenyl)pyridin-3-ylamino)-5-methylbenzoic acid;
65. Ethyl 2-(5-cyclopropyl-6-(3-methoxyphenyl)pyridin-3-ylamino)-5-methylbenzoate;
66. 2-(6-(2-Fluoro-5-isopropoxyphenyl)pyridin-3-ylamino)-5-methylbenzoic acid;
67. 2-(6-(3-Isopropoxyphenyl)-5-methylpyridin-3-ylamino)-5-methylbenzoic acid;
68. Ethyl 2-(6-(3-isopropoxyphenyl)-5-methylpyridin-3-ylamino)-5-methylbenzoate;
69. 2-(6-(3-Cyclopropoxyphenyl)-5-methylpyridin-3-ylamino)-5-methylbenzoic acid;
70. *tert*-Butyl 2-(6-(3-cyclopropoxyphenyl)-5-methylpyridin-3-ylamino)-5-methylbenzoate;
71. 2-(6-(2-Chlorophenyl)-5-methylpyridin-3-ylamino)-5-methylbenzoic acid;
72. *tert*-Butyl 2-(6-(2-chlorophenyl)-5-methylpyridin-3-ylamino)-5-methylbenzoate;
73. 2-(6-(3-Carbamoylphenyl)-5-methylpyridin-3-ylamino)-5-methylbenzoic acid;
74. Ethyl 2-(6-(3-carbamoylphenyl)-5-methylpyridin-3-ylamino)-5-methylbenzoate;
75. 2-(6-(2-Fluoro-5-methoxyphenyl)-4-methylpyridin-3-ylamino)-5-methylbenzoic acid;
76. Ethyl 2-(6-(2-fluoro-5-methoxyphenyl)-4-methylpyridin-3-ylamino)-5-methylbenzoate;
77. 2-(6-(3-Methoxyphenyl)-5-(trifluoromethyl)pyridin-3-ylamino)-5-methylbenzoic acid;
78. Ethyl 2-(6-(3-methoxyphenyl)-5-(trifluoromethyl)pyridin-3-ylamino)-5-methylbenzoate;
79. 2-(6-(3-(Dimethylcarbamoyl)phenyl)-5-methylpyridin-3-ylamino)-5-methylbenzoic acid;
80. Ethyl 2-(6-(3-(dimethylcarbamoyl)phenyl)-5-methylpyridin-3-ylamino)-5-methylbenzoate;
81. 2-(6-(3-Isopropoxyphenyl)-5-methylpyridin-3-ylamino)-3-methylbenzoic acid;
82. *tert*-Butyl 2-(6-(3-isopropoxyphenyl)-5-methylpyridin-3-ylamino)-3-methylbenzoate;
83. 3-Methyl-2-(5-methyl-6-phenylpyridin-3-ylamino)benzoic acid;
84. *tert*-Butyl 3-methyl-2-(5-methyl-6-phenylpyridin-3-ylamino)benzoate;
85. 2-(6-(2-Chlorophenyl)pyridin-3-ylamino)-5-methylbenzoic acid;
86. *tert*-Butyl 2-(6-(2-chlorophenyl)pyridin-3-ylamino)-5-methylbenzoate;
87. 3-Fluoro-2-(6-(3-methoxyphenyl)-5-methylpyridin-3-ylamino)benzoic acid;
88. *tert*-Butyl 3-fluoro-2-(6-(3-methoxyphenyl)-5-methylpyridin-3-ylamino) benzoate;
89. 5-Cyclopropyl-2-(5-methyl-6-(3-(trifluoromethoxy)phenyl)pyridin-3-ylamino)benzoic acid;
90. Ethyl 5-cyclopropyl-2-(5-methyl-6-(3-(trifluoromethoxy)phenyl)pyridin-3-ylamino)benzoate;
91. 5-Cyclopropyl-2-(5-methyl-6-phenylpyridin-3-ylamino)benzoic acid;
92. Ethyl 5-cyclopropyl-2-(5-methyl-6-phenylpyridin-3-ylamino)benzoate;
93. 5-Methyl-2-(5-methyl-6-(2-(trifluoromethyl)phenyl)pyridin-3-ylamino)benzoic acid;
94. *tert*-Butyl 5-methyl-2-(5-methyl-6-(2-(trifluoromethyl)phenyl)pyridin-3-ylamino)benzoate;
95. 2-(6-(3-Chlorophenyl)-5-methylpyridin-3-ylamino)-5-methylbenzoic acid;
96. *tert*-Butyl 2-(6-(3-chlorophenyl)-5-methylpyridin-3-ylamino)-5-methylbenzoate;
97. 2-(6-(2-Fluorophenyl)-5-methylpyridin-3-ylamino)-5-methylbenzoic acid;
98. *tert*-Butyl 2-(6-(2-fluorophenyl)-5-methylpyridin-3-ylamino)-5-methylbenzoate;
99. 5-Methyl-2-(5-methyl-6-(quinolin-5-yl)pyridin-3-ylamino)benzoic acid;
100. *tert*-Butyl 5-methyl-2-(5-methyl-6-(quinolin-5-yl)pyridin-3-ylamino)benzoate;
101. 2-(3'-Fluoro-3-methyl-2,4'-bipyridin-5-ylamino)-5-methylbenzoic acid;
102. *tert*-Butyl 2-(3'-fluoro-3-methyl-2,4'-bipyridin-5-ylamino)-5-methylbenzoate;
103. 5-Methyl-2-(5-methyl-6-(pyrazin-2-yl)pyridin-3-ylamino)benzoic acid;
104. *tert*-Butyl 5-methyl-2-(5-methyl-6-(pyrazin-2-yl)pyridin-3-ylamino)benzoate;
105. 5-Cyclopropyl-2-(6-phenyl-5-(trifluoromethyl)pyridin-3-ylamino)benzoic acid;
106. Ethyl 5-cyclopropyl-2-(6-phenyl-5-(trifluoromethyl)pyridin-3-ylamino)benzoate;
107. 5-Cyclopropyl-2-(6-(3-methoxyphenyl)-5-(trifluoromethyl)pyridin-3-ylamino)benzoic acid;
108. Ethyl 5-cyclopropyl-2-(6-(3-methoxyphenyl)-5-(trifluoromethyl)pyridin-3-ylamino)benzoate;
109. 5-Chloro-2-(6-(2-fluorophenyl)pyridin-3-ylamino)benzoic acid;
110. 5-Chloro-2-(6-(2-chlorophenyl)pyridin-3-ylamino)benzoic acid;
111. 5-Chloro-2-(6-(quinolin-5-yl)pyridin-3-ylamino)benzoic acid;
112. 2-(6-(2-Chlorophenyl)pyridin-3-ylamino)-5-cyclopropylbenzoic acid;
113. Ethyl 2-(6-(2-chlorophenyl)pyridin-3-ylamino)-5-cyclopropylbenzoate;
114. 5-Chloro-2-(6-(2-(trifluoromethyl)phenyl)pyridin-3-ylamino)benzoic acid;
115. 5-Fluoro-2-(6-(2-(trifluoromethyl)phenyl)pyridin-3-ylamino)benzoic acid;
116. 2-(3'-Fluoro-2,4'-bipyridin-5-ylamino)-5-methylbenzoic acid;
117. 2-(2-(2-Fluorophenyl)pyrimidin-5-ylamino)-5-methylbenzoic acid;
118. tert-Butyl 2-(2-(2-fluorophenyl)pyimidin-5-ylamino)-5-methylbenzoate;
119. 2-(6-(2,6-Difluorophenyl)pyridin-3-ylamino)-5-methylbenzoic acid;
120. Ethyl 2-(6-(2,6-difluorophenyl)pyridin-3-ylamino)-5-methylbenzoate;
121. 2-(2-(2-Chlorophenyl)pyrimidin-5-ylamino)-5-cyclopropylbenzoic acid;
122. Methyl 2-(2-(2-chlorophenyl)pyrimidin-5-ylamino)-5-cyclopropylbenzoate;
123. 2-(2-(2-Chlorophenyl)pyrimidin-5-ylamino)-5-methylbenzoic acid;
124. *tert*-Butyl 2-(2-(2-chlorophenyl)pyrimidin-5-ylamino)-5-methylbenzoate ;
125. 5-Methyl-2-(5-methyl-6-(3-(pyrrolidine-1-carbonyl)phenyl)pyridin-3-ylamino)benzoic acid;
126. 2-(6-(3-(Cyclopropylcarbamoyl)phenyl)-5-methylpyridin-3-ylamino)-5-methylbenzoic acid;
127. 5-Cyclopropyl-2-(2-(2-fluorophenyl)pyrimidin-5-ylamino)benzoic acid;
128. 2-(2-(2-trifluoromethylphenyl)pyrimidin-5-ylamino)-5-cyclopropylbenzoic acid;
129. 2-(2-*o*-tolylpyrimidin-5-ylamino)-5-cyclopropylbenzoic acid;
130. 2-(2-(2-cyclopropoxyphenyl)pyrimidin-5-ylamino)-5-cyclopropylbenzoic acid;
131. 2-(2-(2,5-difluorophenyl)pyrimidin-5-ylamino)-5-cyclopropylbenzoic acid;
132. 2-(2-(2,3-difluorophenyl)pyrimidin-5-ylamino)-5-cyclopropylbenzoic acid;
133. 2-(2-(2-fluoro-5-chlorophenyl)pyrimidin-5-ylamino)-5-cyclopropylbenzoic acid;
134. 2-(2-(2-trifluoromethylphenyl)pyrimidin-5-ylamino)-5-methylbenzoic acid;
135. 2-(2-(2-fluoro-5-trifluoromethoxyphenyl)pyrimidin-5-ylamino)-5-cyclopropylbenzoic acid;
136. 2-(6-(2-trifluoromethylphenyl)pyridin-3-ylamino)-5-methylbenzoic acid;
137. 2-(6-phenylpyridin-3-ylamino)-5-cyclopropylbenzoic acid;
138. 2-(6-(2-fluorophenyl)pyridin-3-ylamino)-5-cyclopropylbenzoic acid;
139. 2-(6-(3,5-difluoropyridin-4-yl)pyridin-3-ylamino)-5-methylbenzoic acid;
140. 2-(6-(3-cyclopropylcarbamoylphenyl)pyridin-3-ylamino)-5-methylbenzoic acid;
141. 2-(6-(2,4-difluorophenyl)pyridin-3-ylamino)-5-methylbenzoic acid;
142. 2-(6-(2,5-difluorophenyl)pyridin-3-ylamino)-5-methylbenzoic acid;
143. 2-(6-(2-fluorophenyl)pyridin-3-ylamino)-5-cyclopropyl-3-fluorobenzoic acid;
144. 2-(6-(2,3,6-trifluorophenyl)pyridin-3-ylamino)-5-methylbenzoic acid;
145. 2-(6-(3-(5-methyl-1,3,4-oxadiazol-2-yl)phenyl)pyridin-3-ylamino)-5-methylbenzoic acid;
146. 2-(5-methyl-6-(pyrimidin-5-yl)pyridin-3-ylamino)-5-methylbenzoic acid;
147. 2-(6-(2,3-difluorophenyl)pyridin-3-ylamino)-5-methylbenzoic acid;
148. 2-(6-(5-fluoro-2-methoxyphenyl)-5-methylpyridin-3-ylamino)-5-methylbenzoic acid, and;
149. 2-(6-(4-carbamoylphenyl)-5-methylpyridin-3-ylamino)-5-methylbenzoic acid;
or a pharmaceutically acceptable salt or N-oxide thereof.

More preferably, the DHODH inhibitor is one of:
5-cyclopropyl-2-(2-phenylpyrimidin-5-ylamino)benzoic acid;
5-((2-Carboxy-4-cyclopropylphenylamino)-3-methyl-2-phenylpyridine 1-oxide;
5-Methyl-2-(6-(3-(trifluoromethyl)phenyl)pyridin-3-ylamino)benzoic acid;
2-((5-Fluoro-6-phenylpyridin-3-ylamino)-5-methylbenzoic acid;
5-Cyclopropyl-2-(3'-fluoro-2,4'-bipyridin-5-ylamino)benzoic acid;
2-((5-Chloro-6-phenylpyridin-3-ylamino)-5-methylbenzoic acid;
5-Cyclopropyl-2-(2-(2-cyclopropylphenyl)pyrimidin-5-ylamino)benzoic acid;
5-cyclopropyl-2-(5-phenylpyridin-3-ylamino) benzoic acid;
2-((5-Chloro-2-phenylpyridin-3-ylamino)-5-methylbenzoic acid;
5-Cyclopropyl-2-(5,6-diphenylpyridin-3-ylamino)benzoic acid;
5-cyclopropyl-2-(2-(2,6-difluorophenyl)pyrimidin-5-ylamino)benzoic acid;
2-((2-(3-Cyclopropoxyphenyl)pyrimidin-5-ylamino)-5-cyclopropylbenzoic acid;
5-Methyl-2-(6-morpholinopyridin-3-ylamino)benzoic acid;
5-Methyl-2-(5-methyl-6-morpholinopyridin-3-ylamino)benzoic acid;
5-cyclopropyl-2-(6-cyclopropyl-5-phenylpyridin-3-ylamino) benzoic acid;
2-((6-(2-Cyclopropylphenyl)-5-methylpyridin-3-ylamino)-5-methylbenzoic acid;
2-((6-(2-Cyanophenyl)-5-methylpyridin-3-ylamino)-5-methylbenzoic acid;
2-((2-(3-Chlorophenyl)pyrimidin-5-ylamino)-5-cyclopropylbenzoic acid;
5-Methyl-2-(6-phenyl-5-(trifluoromethyl)pyridin-3-ylamino)benzoic acid;
5-Methyl-2-(5-methyl-6-(piperidin-1-yl)pyridin-3-ylamino)benzoic acid;
2-((6-(Azepan-1-yl)-5-methylpyridin-3-ylamino)-5-methylbenzoic acid;
2-((2,3'-bipyridin-5-ylamino)-5-cyclopropylbenzoic acid;
2-((3'-chloro-2,4'-bipyridin-5-ylamino)-5-methylbenzoic acid;
5-Methyl-2-(3-methyl-2,2'-bipyridin-5-ylamino)benzoic acid;
2-((6-(3-Ethoxyphenyl)-5-methylpyridin-3-ylamino)-5-methylbenzoic acid;
2-((6-(3-Methoxyphenyl)-5-methylpyridin-3-ylamino)-5-methylbenzoic acid;
5-Methyl-2-(5-methyl-6-phenylpyridin-3-ylamino)benzoic acid;
2-((6-(3-Isopropoxyphenyl)-5-methylpyridin-3-ylamino)-5-methylbenzoic acid;
5-Cyclopropyl-2-(5-methyl-6-(3-(trifluoromethoxy)phenyl)pyridin-3-ylamino)benzoic acid;
5-Cyclopropyl-2-(5-methyl-6-phenylpyridin-3-ylamino)benzoic acid;
2-((6-(2-Fluorophenyl)-5-methylpyridin-3-ylamino)-5-methylbenzoic acid;
5-Cyclopropyl-2-(6-phenyl-5-(trifluoromethyl)pyridin-3-ylamino)benzoic acid;
5-Cyclopropyl-2-(6-(3-methoxyphenyl)-5-(trifluoromethyl)pyridin-3-ylamino)benzoic acid;
2-((6-(2-Chlorophenyl)pyridin-3-ylamino)-5-cyclopropylbenzoic acid;
2-((2-(2-Chlorophenyl)pyrimidin-5-ylamino)-5-cyclopropylbenzoic acid;
2-((2-(2-Chlorophenyl)pyrimidin-5-ylamino)-5-methylbenzoic acid;
2-((6-(2,6-Difluorophenyl)pyridin-3-ylamino)-5-methylbenzoic acid ;
5-Methyl-2-(5-methyl-6-(3-(pyrrolidine-1-carbonyl)phenyl)pyridin-3-ylamino)benzoic acid;
2-((6-(3-(Cyclopropylcarbamoyl)phenyl)-5-methylpyridin-3-ylamino)-5-methylbenzoic acid;
5-Cyclopropyl-2-(2-(2-fluorophenyl)pyrimidin-5-ylamino)benzoic acid,
or a pharmaceutically acceptable salt or N-oxide thereof.

Most preferably, the DHODH inhibitor is 5-Methyl-2-(6-(3-(trifluoromethyl)phenyl)pyridin-3-ylamino)benzoic acid, 5-cyclopropyl-2-(2-(2,6-difluorophenyl)pyrimidin-5-ylamino)benzoic acid, 2-(6-(2,6-Difluorophenyl)pyridin-3-ylamino)-5-methylbenzoic acid or 2-(6-(3-(Cyclopropylcarbamoyl)phenyl)-5-methylpyridin-3-ylamino)-5-methylbenzoic acid or a pharmaceutically acceptable salt or N-oxide thereof.

Preferably the active ingredients (a) and (b) form part of a single pharmaceutical composition.

Further provided is a combination for use as described above which further comprises (c) another compound selected from:
(i) Anti-TNF-alpha monoclonal antibodies such as Infliximab, Certolizumab pegol, Golimumab, Adalimumab and AME-527 from Applied Molecular Evolution;
(ii) TNF-alpha Antagonists such as Etanercept, Lenercept, Onercept and Pegsunercept;
(iii) Calcineurin (PP-2B) Inhibitors / INS Expression Inhibitors such as cyclosporine A, Tacrolimus and ISA-247 from Isotechnika;
(iv) IL-1 Receptor Antagonists such as Anakinra and AMG-719 from Amgen;
(v) Anti-CD20 monoclonal antibodies such as Rituximab, Ofatumumab, Ocrelizumab and TRU-015 from Trubion Pharmaceuticals;
(vi) p38 Inhibitors such as AMG-548 (from Amgen), ARRY-797 (from Array Biopharma), Chlormethiazole edisylate, Doramapimod, PS-540446 (from BMS), SB-203580, SB-242235, SB-235699, SB-281832, SB-681323, SB-856553 (all from GlaxoSmithKline), KC-706 (from Kemia), LEO-1606, LEO-15520 (all from Leo), SC-80036, SD-06 (all from Pfizer), RWJ-67657 (from R.W. Johnson), RO-3201195, RO-4402257 (all from Roche), AVE-9940 (from Aventis), SCIO-323, SCIO-469 (all from Scios), TA-5493 (from Tanabe Seiyaku), and VX-745 and VX-702 (all from Vertex);
(vii) NF-kappaB (NFKB) Activation Inhibitors such as Sulfasalazine and Iguratimod;
(viii) Another dihydrofolate reductase (DHFR) inhibitor such as Aminopterin and CH-1504 from Chelsea;
(ix) Janus kinase (JAK) inhibitors, such as CP-690, 550 from Pfizer and INCB-18424, from Incyte;
(x) MEK inhibitor, such as ARRY-162 from Array;
(xi) Sphingosine-1 phosphate receptor agonists, such as fingolimod (Novartis);
(xii) Interferons comprising Interferon beta 1a such as Avonex from Biogen Idec, CinnoVex from CinnaGen and Rebif from Merck Serono, and
(xiii) Interferon beta 1 b such as Betaferon from Schering and Betaseron from Berlex;
(xiv) Inmunomodulators suchs as BG-12 (fumaric acid derivative) from Biogen Idec/Fumapharm AG; laquinimod (Teva and Active Biotech) or glatiramer acetate (Teva); and
(xv) Adenosine aminohydrolase inhibitors such as Cladribine from Merck Serono.

The present invention also provides a combination comprising (a) methotrexate and (b) a DHODH inhibitor which is 5-methyl-2-(6-(3-(trifluoromethyl)phenyl)pyridin-3-ylamino)benzoic acid; 5-cyclopropyl-2-(2-(2,6-difluorophenyl)pyrimidin-5-ylamino)benzoic acid; 2-(6-(2,6-difluorophenyl)pyridin-3-ylamino)-5-methylbenzoic acid; 2-(6-(3-(cyclopropylcarbamoyl)phenyl)-5-methylpyridin-3-ylamino)-5-methylbenzoic acid; 5-cyclopropyl-2-(2-(2,6-difluorophenyl)pyrimidin-5-ylamino)benzoic acid or a pharmaceutically acceptable salt or N-oxide thereof.

Diseases or disorders in which DHODH inhibition plays a role include without limitation autoimmune diseases, immune and inflammatory diseases, destructive bone disorders, malignant neoplastic diseases, angiogenic-related disorders, viral diseases, and infectious diseases.

Autoimmune diseases which may be prevented or treated include but are not limited to rheumatoid arthritis, psoriatic arthritis, systemic lupus erythematosus, multiple sclerosis, psoriasis, ankylosing spondilytis, Wegener's granulomatosis, polyarticular juvenile idiopathic arthritis, inflammatory bowel disease such as ulcerative colitis and Crohn's disease, Reiter's syndrome, fibromyalgia and type-1 diabetes.

Immune and inflammatory diseases which may be prevented or treated include but are not limited to asthma, COPD, respiratory distress syndrome, acute or chronic pancreatitis, graft versus-host disease, chronic sarcoidosis, transplant rejection, contact dermatitis, atopic dermatitis, allergic rhinitis, allergic conjunctivitis, Behcet syndrome, inflammatory eye conditions such as conjunctivitis and uveitis.

Destructive bone disorders which may be prevented or treated include but are not limited to osteoporosis, osteoarthritis and multiple myeloma-related bone disorder.

Malignant neoplastic diseases that may be prevented or treated include but are not limited to prostate, ovarian and brain cancer.

Angiogenesis-related disorders that may be prevented or treated include but are not limited to haemangioma, ocular neovascularization, macular degeneration or diabetic retinopathy.

Viral diseases which may be prevented or treated include but are not limited to HIV infection, hepatitis and cytomegalovirus infection.

Infectious diseases which may be prevented or treated include but are not limited to sepsis, septic shock, endotoxic shock, Gram negative sepsis, toxic shock syndrome, Shigellosis and other protozoal infestations such as malaria.

Preferably, the pathological condition or disease is selected from rheumatoid arthritis, psoriatic arthritis, ankylosing spondilytis, multiple sclerosis, Wegener's granulomatosis, systemic lupus erythematosus, psoriasis and sarcoidosis. More preferably the pathological condition or disease is rheumatoid arthritis, psoriatic arthritis or psoriasis. Most preferably it is rheumatoid arthritis.

Also disclosed is a combination comprising (a) Interferons such as Interferon beta 1a or Interferon beta 1b, and (b) a DHODH inhibitor of the invention, preferably a DHODH inhibitor of formula (I).

Also disclosed is the use of a combination comprising (a) Interferons such as Interferon beta 1 a or Interferon beta 1b, and (b) a DHODH inhibitor of the invention, preferably a DHODH inhibitor of formula (I) for the preparation of a medicament for simultaneous, separate or sequential use for the treatment of multiple scleroris.

Also provided is a product comprising (a) methotrexate and (b) a DHODH inhibitor of the invention, as a combined preparation for simultaneous, separate or sequential use in the treatment of a human or animal patient suffering from or susceptible to a pathological condition or disease as defined above
wherein
(i) the methotrexate (a) is for administration at a dosage regime which involves administration of 0.015 to 3 mg/kg/week of methotrexate and the DHODH inhibitor (b) is for administration at a dosage regime which involves administration of 0.03 to 30 mg/kg/day of DHODH inhibitor; or
(ii) the combination is for use in treating a human or animal patient suffering from hepatic impairment, liver fibrosis, hepatitis, cirrhosis or liver cancer.

Said product may optionally further comprise an active compound (c), as defined above.

Also provided is a kit of parts comprising (b) a DHODH inhibitor of the invention together with instructions for simultaneous, separate or sequential use in combination with (a) methotrexate, for the treatment of a human or animal patient suffering from or susceptible to a pathological condition or disease as defined above
wherein
(i) the methotrexate (a) is for administration at a dosage regime which involves administration of 0.015 to 3 mg/kg/week of methotrexate and the DHODH inhibitor (b) is for administration at a dosage regime which involves administration of 0.03 to 30 mg/kg/day of DHODH inhibitor; or
(ii) the combination is for use in treating a human or animal patient suffering from hepatic impairment, liver fibrosis, hepatitis, cirrhosis or liver cancer.

Said kit may optionally further comprise an active compound (c), as defined above.

Also provided is a package comprising (b) a DHODH inhibitor of the invention and (a) methotrexate, for simultaneous, separate or sequential use in the treatment of a pathological condition or disease as defined above
wherein
(i) the methotrexate (a) is for administration at a dosage regime which involves administration of 0.015 to 3 mg/kg/week of methotrexate and the DHODH inhibitor (b) is for administration at a dosage regime which involves administration of 0.03 to 30 mg/kg/day of DHODH inhibitor; or
(ii) the combination is for use in treating a human or animal patient suffering from hepatic impairment, liver fibrosis, hepatitis, cirrhosis or liver cancer.

Said package may optionally further comprise an active compound (c), as defined above.

Also disclosed is a use of (b) a DHODH inhibitor of the invention for the preparation of a medicament, for use in combination with (a) methotrexate, for the treatment of a pathological condition or disease as defined above.

Also disclosed is a use of (a) methotrexate, for the preparation of a medicament, for use in combination with (b) a DHODH inhibitor of the invention, for the treatment of a pathological condition or disease as defined above.

As mentioned above, the methotrexate (a) may be for administration at a dosage regime which involves administration of 0.015 to 3 mg/kg/week of methotrexate and the DHODH inhibitor (b) may be for administration at a dosage regime which involves administration of 0.03 to 30 mg/kg/day of DHODH inhibitor.

Typically the medicament is for use in treating a human or animal patient suffering from hepatic impairment or a condition that would be aggravated by hepatotoxicity. More typically, the said human or animal patient is suffering from liver fibrosis, hepatitis (typically hepatitis A to G), cirrhosis (typically caused by alcoholism) or liver cancer.

In one embodiment of the present invention, the combination, product, kit of parts or package comprises (b) a DHODH inhibitor of the invention, and (a) methotrexate, as the sole active components.

The fact that the DHODH inhibitors of the invention have reduced hepatic side effects is a finding of the invention. Also disclosed herein is the use of a DHODH inhibitor of the invention, as defined above, in the manufacture of a medicament for use in treating or preventing a pathological condition or disease, as defined above, in a human or animal patient which is suffering from or susceptible to hepatic impairment or a condition that would be aggravated by hepatotoxicity, as defined above.

Also disclosed is a method of treating a human or animal patient suffering from or susceptible to a pathological condition or disease as defined above, which method comprises simultaneously, separately or sequentially administering to said human or animal patient a therapeutically effective amount of (a) methotrexate and (b) a DHODH inhibitor as defined above. Preferably in said method, (a) methotrexate and (b) the DHODH inhibitor are the sole active components.

Also disclosed is a method of treating a human or animal patient suffering from or susceptible to a pathological condition or disease as defined above, wherein the human or animal patient is suffering from or susceptible to hepatic impairment or a condition that would be aggravated by hepatotoxicity as defined above, which method comprises administering to said human or animal patient a therapeutically effective amount of a DHODH inhibitor as defined above.

The active compounds in the combinations of the invention may be administered by any suitable route, depending on the nature of the disorder to be treated, e.g. orally (as syrups, tablets, capsules, lozenges, controlled-release preparations, fast-dissolving preparations, etc); topically (as creams, ointments, lotions, nasal sprays or aerosols, etc); by injection (subcutaneous, intradermic, intramuscular, intravenous, etc.) or by inhalation (as a dry powder, a solution, a dispersion, etc).

The active compounds in the combination may be administered together in the same pharmaceutical composition or in different compositions intended for separate, simultaneous, concomitant or sequential administration by the same or a different route.

The combinations of the invention may conveniently be presented in unit dosage form and may be prepared by any of the methods well known in the art of pharmacy.

Combinations of the present invention suitable for oral administration may be presented as discrete units such as capsules, cachets or tablets each containing a predetermined amount of the active ingredient; as a powder or granules; as a solution or a suspension in an aqueous liquid or a non-aqueous liquid; or as an oil- in-water liquid emulsion or a water-in-oil liquid emulsion. The active ingredient may also be presented as a bolus, electuary or paste.

A syrup formulation will generally consist of a suspension or solution of the compound or salt in a liquid carrier for example, ethanol, peanut oil, olive oil, glycerine or water with flavouring or colouring agent.

Where the combination is in the form of a tablet, any pharmaceutical carrier routinely used for preparing solid formulations may be used. Examples of such carriers include magnesium stearate, talc, gelatine, acacia, stearic acid, starch, lactose and sucrose.

A tablet may be made by compression or moulding, optionally with one or more accessory ingredients. Compressed tablets may be prepared by compressing in a suitable machine the active ingredient in a free-flowing form such as a powder or granules, optionally mixed with a binder, lubricant, inert diluent, lubricating, surface active or dispersing agent. Moulded tablets may be made by moulding in a suitable machine a mixture of the powdered compound moistened with an inert liquid diluent. The tablets may optionally be coated or scored and may be formulated so as to provide slow or controlled release of the active ingredient therein.

Where the combination is in the form of a capsule, any routine encapsulation is suitable, for example using the aforementioned carriers in a hard gelatine capsule. Where the composition is in the form of a soft gelatine capsule any pharmaceutical carrier routinely used for preparing dispersions or suspensions may be considered, for example aqueous gums, celluloses, silicates or oils, and are incorporated in a soft gelatine capsule.

The combination may be in the form of a dry powder composition for topical delivery to the lung by inhalation. Dry powder compositions may, for example, be presented in capsules and cartridges of for example gelatine or blisters of for example laminated aluminium foil, for use in an inhaler or insufflator. Formulations generally contain a powder mix for inhalation of the compound of the invention and a suitable powder base (carrier substance) such as lactose or starch. Use of lactose is preferred. Each capsule or cartridge may generally contain between 2 µg and 150 µg of each therapeutically active ingredient. Alternatively, the active ingredient (s) may be presented without excipients.

Packaging of the formulation for inhalation may be carried out by using suitable inhaler devices such as the Genuair^{®} (formerly known as Novolizer SD2FL) which is described in the following patent applications: WO 97/000703, WO 03/000325 and WO 03/061742.

The combination may be in the form of a composition for nasal delivery. Typical compositions for nasal delivery include those mentioned above for inhalation and further include non-pressurized compositions in the form of a solution or suspension in an inert vehicle such as water optionally in combination with conventional excipients such as buffers, anti-microbials, tonicity modifying agents and viscosity modifying agents which may be administered by nasal pump.

Typical dermal and transdermal formulations comprise a conventional aqueous or non-aqueous vehicle, for example a cream, ointment, lotion or paste or are in the form of a medicated plaster, patch or membrane.

Preferably the combination is in unit dosage form, for example a tablet, capsule or metered aerosol dose, so that the patient may administer a single dose.

The amount of each active which is required to achieve a therapeutic effect will, of course, vary with the particular active, the route of administration, the subject under treatment, and the particular disorder or disease being treated.

Typically all active agents in the combination are administered at the same time, or very close in time. Alternatively, one or two actives could be taken in the morning and the other (s) later in the day. Or in another scenario, one or two actives could be taken twice daily and the other (s) once daily, either at the same time as one of the twice-a-day dosing occurred, or separately. Preferably at least two, and more preferably all, of the actives would be taken together at the same time. Preferably, at least two, and more preferably all actives would be administered as an admixture.

Preferably the drug combination of the invention is for administration as a dosage regime which involves administration of (i) 0.015 to 3 mg/kg/week of methotrexate, more preferably 0.07 to 0.7 mg/kg/week of methotrexate and most preferably 0.15 to 0.35 mg/kg/week of methotrexate, and (ii) 0.03 to 30 mg/kg/day of DHODH inhibitor, more preferably 0.07 to 14 mg/day of DHODH inhibitor and most preferably 0.15 to 0.3 mg/kg/day of DHODH inhibitor.

### EXAMPLES

### Example 1 - inhibition of human DHODH activity assay

DHODH activity and its inhibition were studied using a chromogen reduction assay with DCIP (2,6-dichlorophenol-indophenol). The substrate oxidation (Dihydroorotate, L-DHO), as well as co-substrate reduction (coenzyme Q, CoQ) is coupled to the chromogen reduction, hence enzymatic activity results in a loss of chromogen absorbance at 600 nm.

Enzyme extracts (8 µl, ~1.5 µg of human protein) were incubated in 96-well plates. The assay mixture (200 µl) contained 200 µM CoQD, 100 µM L-DHO, 120 µM DCIP in the assay buffer (100 mM HEPES pH 8.0, 150 mM NaCl, 10% Glicerol, 0.05% Triton X-100) and 2 µl of test compound. The compounds were dissolved in DMSO at a stock concentration of 1 mM, and tested at different concentrations varying from 10 µM to 1 pM to calculate an IC₅₀ (concentration of inhibitor required for 50% of inhibition).

The reaction was initiated by adding the enzyme and then incubated for 10 min at room temperature before measuring DCIP reduction by counting a decrease in absorbance at 600 nm using standard instrumentation (Spectramax).

All reactions were carried out in duplicate and graphs, determining IC₅₀ values for each compound, were plotted using the ABase software.

Table 1 shows the activities in human DHODH inhibition assay of some compounds of the present invention (compounds from the list indicated previously) showing that these compounds are potent DHODH inhibitors.

**TABLE 1**

| **Compound No.** | **hDHODH IC₅₀ (nM)** |
|---|---|
| 1 | 105 |
| 4 | 98 |
| 6 | 57 |
| 7 | 49 |
| 8 | 18 |
| 14 | 57 |
| 15 | 113 |
| 18 | 62 |
| 19 | 10 |
| 20 | 14 |
| 22 | 114 |
| 25 | 28 |
| 30 | 41 |
| 31 | 119 |
| 34 | 109 |
| 41 | 190 |
| 42 | 30 |
| 44 | 78 |
| 50 | 138 |
| 51 | 21 |
| 54 | 19 |
| 57 | 91 |
| 60 | 53 |
| 66 | 28 |
| 67 | 11 |
| 71 | 14 |
| 73 | 190 |
| 75 | 97 |
| 77 | 12 |
| 79 | 33 |
| 85 | 32 |
| 89 | 5 |
| 91 | 6 |
| 93 | 20 |
| 95 | 10 |
| 97 | 5 |
| 101 | 37 |
| 105 | 2 |
| 107 | 7 |
| 110 | 145 |
| 112 | 4 |
| 116 | 90 |
| 119 | 19 |
| 121 | 3 |
| 123 | 57 |
| 125 | 9 |
| 126 | 12 |
| 127 | 10 |
| 128 | 9 |
| 129 | 12 |
| 131 | 38 |
| 135 | 21 |
| 138 | 8 |
| 141 | 146 |
| 144 | 77 |
| 148 | 46 |

### Example 2 - reduced hepatotoxicity

Acute hepatotoxicity assays were performed in Swiss mice. Animals received a single administration of either vehicle, or 100 mg/kg of teriflunomide or a compound of the present invention (compounds from the list indicated previously) by intraperitoneal route. Twenty-four hours later, animals were sacrificed and the levels of liver markers AST (aspartate aminotransferase), ALT (alanine aminotransferase) and BIL (total bilirubin) in plasma were determined.

**Table 2: Plasma levels of liver markers of mice after administration of 100 mg/kg of the compound. 100 mg/kg Teriflunomide or vehicle (IU: International Units).**

| **Compound No.** | ALT (IU/l) | AST (IU/l) | BIL (mg/dl) |
|---|---|---|---|
| **20** | 45 | 98 | 0.07 |
| **60** | 70 | 131 | 0.09 |
| **71** | 72 | 95 | 0.05 |
| **85** | 43 | 83 | 0.13 |
| **97** | 55 | 92 | 0.11 |
| **119** | 69 | 96 | 0.08 |
| **121** | 75 | 105 | 0.05 |
| **123** | 89 | 113 | 0.06 |
| **127** | 56 | 72 | 0.07 |
| **Vehicle** | 68 | 92 | 0.1 |
| **Teriflunomide** | 440 | 655 | 0.46 |

As it can clearly seen from Table 2, Teriflunomide-treated mice showed a dramatic increase in the three liver markers compared to vehicle-treated mice, clearly indicating a high hepatotoxicity, whereas the DHODH inhibitors according to the present invention did not cause a significant increase in any of the parameters measured

### Example 3: Efficacy assay in adjuvant-induced arthritis of the combination product of the present invention.

The effect of DHODH inhibitor compounds were tested in combination with methotrexate (0.05 mg/Kg/ day) in the rat adjuvant-induced arthritis model (AIA) in animals with established disease (curative protocol). Briefly, Complete Freund Adjuvant (CFA) was injected into the left hind footpad of Wistar rats, and 10 days later the swelling of the two rear paws was measured with a plethysmometer. Rats exhibiting a similar degree of inflammation in both paws were randomized into treatment groups (n=7 per group). Compounds were administered orally once a day for 10 days and paw volumes were determined every two days up to day 21.

**Table 3. Effects of compound 20 (10 mg/Kg/day), Methotrexate (0.05 mg/Kg/day) and their combination on the inhibition of paw inflammation in arthritic rats.**

| | |
|---|---|
| Results are expressed as the percentage of inhibition of the area under the curve (AUC) of the paw inflammation (measured by plethysmometry) in the period comprised between days 10 and 21 post-induction of arthritis. The percentage of inhibition for every group was calculated versus values from vehicle-treated rats. The table below depicts the mean and SEM of one experiment using 6 animals per group. | |

| **Treatment** | **% inhibition of the inflammation (AUC) Right paw** |
|---|---|
| Compound 20 (10 mg/Kg) | 32 ± 8 |
| MTX (0.05 mg/Kg) | 33 ± 9 |
| Compound 20 (10 mg/Kg) + MTX (0.05 mg/Kg) | 53 ± 6 |

Results from Table 3 show that Compound 20 of the present invention inhibits the inflammation caused by experimental arthritis in rats. Furthermore, the co-administration of MTX and compound 20 resulted in an increased efficacy (65%) versus compound 20 alone, thus indicating the feasibility of administering the compound in patients treated with MTX.

## Claims

1. A combination for simultaneous, separate or sequential use in the treatment of a pathological condition or disease susceptible to amelioration by inhibition of dihydroorotate dehydrogenase which is selected from rheumatoid arthritis, psoriatic arthritis, ankylosing spondilytis, multiple sclerosis, Wegener's granulomatosis, systemic lupus erythematosus, psoriasis and sarcoidosis, which comprises (a) methotrexate and (b) a non-hepatotoxic DHODH inhibitor of formula (I): 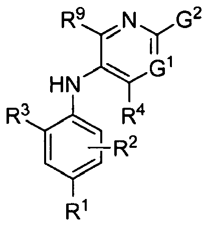 wherein:
R¹ is selected from the group consisting of hydrogen atoms, halogen atoms, C₁₋₄ alkyl, C₃₋₄ cycloalkyl, -CF₃ and -OCF₃,
R² is selected from the group consisting of hydrogen atoms, halogen atoms and C₁₋₄ alkyl groups,
R³ is selected from the group consisting of -COOR⁵, -CONHR⁵, tetrazolyl, -SO₂NHR⁵ and -CONHSO₂R⁵ groups, wherein R⁵ is selected from the group consisting of a hydrogen atom and linear or branched C₁₋₄ alkyl groups,
R⁴ is selected from the group consisting of a hydrogen atom and a C₁₋₄ alkyl group,
R⁹ is selected from the group consisting of a hydrogen atom and a phenyl group,
G¹ represents a group selected from N and CR⁶ wherein R⁶ is selected from the group consisting of hydrogen atoms, halogen atoms, C₁₋₄ alkyl, C₃₋₄ cycloalkyl, C₁₋₄ alkoxy, -CF₃, -OCF₃, monocyclic N-containing C₅₋₇ heteroaryl, monocyclic N-containing C₃₋₇ heterocyclyl groups and C₆₋₁₀ aryl groups which C₆₋₁₀ aryl groups are optionally substituted with one or more substituents selected from halogen atoms and C₁₋₄ alkyl groups,
G² represents a group selected from:
• a hydrogen atom, a hydroxy group, a halogen atom, a C₃₋₄ cycloalkyl group, a C₁₋₄ alkoxy group and -NR^{a}R^{b}, wherein
R^{a} represents a C₁₋₄ alkyl group and R^{b} is selected from a group consisting of C₁₋₄ alkyl group and C₁₋₄ alkoxy-C₁₋₄ alkyl group, or
R^{a} and R^{b} together with the nitrogen atom to which they are attached form a saturated 6 to 8 membered heterocyclic ring optionally containing one oxygen atom as an additional heteroatom,
• a monocyclic or bicyclic 5 to 10 membered heteroaromatic ring containing one or more nitrogen atoms which is optionally substituted by one or more substituents selected from halogen atoms, C₁₋₄ alkyl, C₁₋₄ alkoxy, C₃₋₄ cycloalkyl, C₃₋₄ cycloalkoxy, -CF₃, -OCF₃, and -CONR⁷R⁸, wherein R⁷ and R⁸ are independently selected from hydrogen atom, linear or branched C₁₋₄ alkyl groups, C₃₋₇ cycloalkyl groups, or R⁷ and R⁸ together with the nitrogen atom to which they are attached form a group of formula 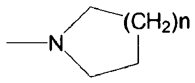 wherein n is an integer from 0 to 3,
and
• a phenyl group which is optionally substituted by one or more substituents selected from halogen atoms, C₁₋₄ alkyl, hydroxyl, C₁₋₄ alkoxy, C₃₋₄cycloalkyl, C₃₄ cycloalkoxy, cyano, -CF₃, -OCF₃, -CONR⁷R⁸, oxadiazolyl, triazolyl, pyrazolyl and imidazolyl groups, which oxadiazolyl, triazolyl, pyrazolyl and imidazolyl groups are optionally substituted by C₁₋₄ alkyl or C₃₋₇ cycloalkyl groups and wherein R⁷ and R⁸ are independently selected from hydrogen atom, linear or branched C₁₋₄ alkyl groups, C₃₋₇ cycloalkyl groups, or R⁷ and R⁸ together with the nitrogen atom to which they are attached form a group of formula 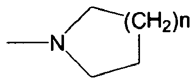 wherein n is an integer from 0 to 3
or, when G¹ represents CR⁶, G² together with R⁶ forms a non-aromatic C₅₋₁₀ carbocyclic group or a C₆₋₁₀ aryl group,
or a pharmaceutically acceptable salts or N-oxide thereof,
wherein
(i) the methotrexate (a) is for administration at a dosage regime which involves administration of 0.015 to 3 mg/kg/week of methotrexate and the DHODH inhibitor (b) is for administration at a dosage regime which involves administration of 0.03 to 30 mg/kg/day of DHODH inhibitor; or
(ii) the combination is for use in treating a human or animal patient suffering from hepatic impairment, liver fibrosis, hepatitis, cirrhosis or liver cancer.

2. A combination for use according to claim 1, wherein:
R¹ is selected from the group consisting of hydrogen atoms, fluorine atoms, chlorine atoms, bromine atoms, C₁₋₄ alkyl, C₃₋₄ cycloalkyl, and -CF₃ groups;
R² is selected from the group consisting of hydrogen atoms, halogen atoms and a methyl group;
R³ is selected from the group consisting of -COOR⁵, -CONHR⁵, tetrazolyl, -SO₂NHR⁵ and -CONHSO₂R⁵ groups, wherein R⁵ is selected from the group consisting of a hydrogen atom and linear or branched C₁₋₄ alkyl groups,
R⁴ is selected from the group consisting of a hydrogen atom and a C₁₋₄ alkyl group,
R⁹ is selected from the group consisting of a hydrogen atom and a phenyl group,
G¹ is selected from the group consisting of nitrogen atoms, CCl, CF, CH, C(CH₃), C(cyclopropyl), C(phenyl) and C(CF₃) groups; and/or
G² represents a group selected from:
• a hydrogen atom, a halogen atom, a C₃₋₄ cycloalkyl group, a C₁₋₂ alkoxy group and -NR^{a}R^{b}, wherein
R^{a} represents a C₁₋₂ alkyl group and R^{b} is selected from the group consisting of C₁₋₂ alkyl groups and C₁₋₂ alkoxy-C₁₋₂ alkyl groups, or
R^{a} and R^{b} together with the nitrogen atom to which they are attached form a saturated 6 or 7 membered heterocyclic ring optionally containing one oxygen atom as an additional heteroatom,
• a monocyclic or bicyclic 5 to 10 membered heteroaromatic ring containing one or two nitrogen atoms which is optionally substituted by one or more substituents selected from halogen atoms and C₁₋₄ alkyl groups,
and
• a phenyl group which is optionally substituted by one, two or three substituents selected from halogen atoms, C₁₋₄ alkyl, hydroxyl, C₁₋₄ alkoxy, C₃₋₄cycloalkyl, C₃₄ cycloalkoxy, cyano, -CF₃, -OCF₃, -CONR⁷R⁸ and oxadiazolyl groups, which oxadiazolyl groups are optionally substituted by a C₁₋₄ alkyl or a C₃₋₇ cycloalkyl group and wherein R⁷ and R⁸ are independently selected from hydrogen atoms, linear or branched C₁₋₄ alkyl groups, C₃₋₄ cycloalkyl groups, or R⁷ and R⁸ together with the nitrogen atom to which they are attached form a group of formula 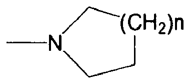
wherein n is 1 or 2,
or, G¹ and G² together form a non-aromatic C₆ carbocyclic group or a phenyl group;
or a pharmaceutically acceptable salt or N-oxide thereof.

3. A combination for use according to any claim 1 or 2, wherein
(i) G² represents a group selected from:
• a hydrogen atom, a fluorine atom, a cyclopropyl group, a methoxy group, -NMeEt, -NEt₂, -N(Me)-(CH₂)₂-O-CH₃, 6-morpholinyl, azepan-1-yl and piperidin-1-yl,
• a pyridinyl, pyrimidinyl, quinolinyl or pyrazinyl ring optionally substituted with one or two substituents selected from Me and F
and
• a phenyl group which is optionally substituted by one, two or three substituents selected from fluorine, chlorine, methyl, hydroxyl, methoxy, ethoxy, isopropyloxy, cyclopropyl, cyclopropyloxy, cyano, -CF₃, -OCF₃ ,oxadiazolyl and -CONR⁷R⁸ groups, which oxadiazolyl groups are optionally substituted by a methyl group and wherein R⁷ and R⁸ are independently selected from hydrogen atom, methyl group, isopropyl group, cyclopropyl group, or R⁷ and R⁸ together with the nitrogen atom to which they are attached form a group of formula 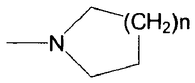
wherein n is 1,
or, G¹ and G² together form a non-aromatic C₆ carbocyclic group or a phenyl group; and/or
(ii) G² represents a group selected from methoxy group, cyclopropyl group and optionally substituted phenyl, pyridyl, quinolynyl, pyrimidinyl and pyrazinyl groups.

4. A combination for use according to any one of claims 1 to 3, wherein:
(i) R¹ is selected from the group consisting of C₁₋₄ alkyl, C₃₋₄ cycloalkyl and -CF₃ groups, typically selected from the group consisting of methyl and cyclopropyl groups, preferably a cyclopropyl group; and or
(ii) R² is selected from a hydrogen or halogen atom, typically a hydrogen atom; and/or
(iii) R³ is selected from the group consisting of COOR⁵, -CONHR⁵ and tetrazolyl groups, typically a -COOH group; and/or
(iv) R⁴ represents a hydrogen atom or a methyl group, typically a hydrogen atom; and/or
(v) R⁹ represents a hydrogen atom; and/or
(vi) G¹ is selected from the group consisting of nitrogen atoms and CH, C(CH₃), C(cyclopropyl), C(phenyl) and C(CF₃) groups; and/or
(vii) G² represents a group selected from optionally substituted phenyl, pyridyl, quinolynyl, pyrimidinyl and pyrazinyl groups, typically selected from the group consisting of an optionally substituted phenyl, 4-pyridyl, 5-quinolynyl and 2-pyrazinyl groups.

5. A combination for use according to claim 1 or 2, wherein R¹ is selected from a methyl or cyclopropyl group, R² represents a hydrogen atom, R³ is a COOH group, R⁴ represents a hydrogen atom or a methyl group, G¹ is selected from nitrogen atoms and CH, C(CH₃), C(cyclopropyl), C(phenyl) and C(CF₃) groups and G² represents a group selected from the group consisting of an optionally substituted phenyl, 4-pyridyl, 5-quinolynyl and 2-pyrazinyl groups; and wherein R⁹ optionally represents a hydrogen atom.

6. A combination for use according to claim 1 or 2, wherein R¹ is selected from a methyl or cyclopropyl group, R² represents a hydrogen atom, R³ is a COOH group, R⁴ represents a hydrogen atom, G¹ is selected from nitrogen atoms and CH, C(CH₃) and C(CF₃) groups and G² represents a phenyl group optionally substituted with one or two substituents selected from chloro, fluoro, methoxy, ethoxy, isopropoxy, trifluoromethoxy and -CONR⁷R⁸, wherein R⁷ is hydrogen and R⁸ is cyclopropyl or R⁷ and R⁸ together with the nitrogen atom to which they are attached form a group of formula 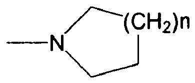 wherein n is 1.

7. A combination for use according to claim 1, wherein the DHODH inhibitor is one of:
5-cyclopropyl-2-(2-phenylpyrimidin-5-ylamino)benzoic acid;
2-((6-Cyclopropyl-5-methylpyridin-3-ylamino)-5-methylbenzoic acid;
5-((2-Carboxy-4-cyclopropylphenylamino)-3-methyl-2-phenylpyridine 1-oxide;
5-Methyl-2-(6-(3-(trifluoromethyl)phenyl)pyridin-3-ylamino)benzoic acid;
5-cyclopropyl-2-(2-(2,6-difluoro-4-hydroxyphenyl)pyrimidin-5-ylamino)benzoic acid;
5-Cyclopropyl-2-(6-methoxy-5-phenylpyridin-3-ylamino) benzoic acid;
2-((5-Fluoro-6-phenylpyridin-3-ylamino)-5-methylbenzoic acid;
2-((6-(Ethyl(methyl)amino)-5-methylpyridin-3-ylamino)-5-methylbenzoic acid;
5-Cyclopropyl-2-(3'-fluoro-2,4'-bipyridin-5-ylamino)benzoic acid;
2-((6-(Diethylamino)-5-methylpyridin-3-ylamino)-5-methylbenzoic acid;
2-((6-((2-Methoxyethyl)(methyl)amino)-5-methylpyridin-3-ylamino)-5-methylbenzoic acid;
2-((5-Chloro-6-phenylpyridin-3-ylamino)-5-methylbenzoic acid;
5-Cyclopropyl-2-(2-(2-cyclopropylphenyl)pyrimidin-5-ylamino)benzoic acid;
5-cyclopropyl-2-(5-phenylpyridin-3-ylamino) benzoic acid;
5-methyl-2-(quinolin-3-ylamino)benzoic acid;
5-methyl-2-(5,6,7,8-tetrahydroquinolin-3-ylamino)benzoic acid;
2-((5-Chloro-2-phenylpyridin-3-ylamino)-5-methylbenzoic acid;
5-Cyclopropyl-2-(5,6-diphenylpyridin-3-ylamino)benzoic acid;
5-cyclopropyl-2-(2-(2,6-difluorophenyl)pyrimidin-5-ylamino)benzoic acid;
5-Cyclopropyl-2-(5-methylpyridin-3-ylamino) benzoic acid;
2-((2-(3-Cyclopropoxyphenyl)pyrimidin-5-ylamino)-5-cyclopropylbenzoic acid;
5-cyclopropyl-2-(6-cyclopropyl-5-phenylpyridin-3-ylamino) benzoic acid;
2-((6-(2-Cyclopropylphenyl)-5-methylpyridin-3-ylamino)-5-methylbenzoic acid;
2-((6-(2-Cyanophenyl)-5-methylpyridin-3-ylamino)-5-methylbenzoic acid;
2-((2-(3-Chlorophenyl)pyrimidin-5-ylamino)-5-cyclopropylbenzoic acid;
5-Methyl-2-(6-phenyl-5-(trifluoromethyl)pyridin-3-ylamino)benzoic acid;
2-((6-(3-Methoxyphenyl)-5-phenylpyridin-3-ylamino)-5-methylbenzoic acid;
2-((2,3'-bipyridin-5-ylamino)-5-cyclopropylbenzoic acid;
2-((3'-chloro-2,4'-bipyridin-5-ylamino)-5-methylbenzoic acid;
5-Methyl-2-(3-methyl-2,2'-bipyridin-5-ylamino)benzoic acid;
2-((5,6-Difluoropyridin-3-ylamino)-5-methylbenzoic acid;
2-((6-(3-Methoxyphenyl)pyridin-3-ylamino)benzoic acid;
2-((6-(3-Ethoxyphenyl)pyridin-3-ylamino)benzoic acid;
2-((6-(3-Ethoxyphenyl)pyridin-3-ylamino)-5-fluorobenzoic acid;
2-((6-(3-Ethoxyphenyl)-5-methylpyridin-3-ylamino)benzoic acid;
2-((6-(3-Ethoxyphenyl)pyridin-3-ylamino)-5-methylbenzoic acid;
2-((6-(3-Ethoxyphenyl)-5-methylpyridin-3-ylamino)-5-methylbenzoic acid;
2-((6-(3-Ethoxy-2-fluorophenyl)pyridin-3-ylamino)benzoic acid;
2-((6-(3-Ethoxyphenyl)-4-methylpyridin-3-ylamino)-5-methylbenzoic acid;
2-((6-(3-Ethoxyphenyl)-4-methylpyridin-3-ylamino)benzoic acid;
5-Bromo-2-(6-(3-ethoxyphenyl)pyridin-3-ylamino)benzoic acid;
5-Chloro-2-(6-(3-ethoxyphenyl)pyridin-3-ylamino)benzoic acid;
2-((6-(5-Ethoxy-2-fluorophenyl)pyridin-3-ylamino)benzoic acid;
2-((6-(3-Ethoxyphenyl)-5-methylpyridin-3-ylamino)-5-(trifluoromethyl)benzoic acid;
2-((6-(3-Methoxyphenyl)-5-methylpyridin-3-ylamino)-5-(trifluoromethyl)benzoic acid;
2-((6-(3-Methoxyphenyl)-5-methylpyridin-3-ylamino)-5-methylbenzoic acid;
2-((6-(3-Methoxyphenyl)-5-methylpyridin-3-ylamino)-6-methylbenzoic acid;
5-Fluoro-2-(6-(3-methoxyphenyl)-5-methylpyridin-3-ylamino)benzoic acid;
2-((6-(5-Ethoxy-2-fluorophenyl)pyridin-3-ylamino)-5-methylbenzoic acid;
2-((6-(2-Fluoro-5-methoxyphenyl)-5-methylpyridin-3-ylamino)-5-methylbenzoic acid;
Ethyl 2-(6-(2-fluoro-5-methoxyphenyl)-5-methylpyridin-3-ylamino)-5-methylbenzoate;
2-((6-(2-Fluorophenyl)pyridin-3-ylamino)-5-methylbenzoic acid;
2-((6-(3-Methoxyphenyl)-5-phenylpyridin-3-ylamino)-5-methylbenzoic acid;
Ethyl 2-(6-(3-methoxyphenyl)-5-phenylpyridin-3-ylamino)-5-methylbenzoate;
5-Methyl-2-(5-methyl-6-phenylpyridin-3-ylamino)benzoic acid;
Ethyl 5-methyl-2-(5-methyl-6-phenylpyridin-3-ylamino)benzoate;
5-Methyl-2-(5-methyl-6-(3-(trifluoromethoxy)phenyl)pyridin-3-ylamino)benzoic acid;
Ethyl 5-methyl-2-(5-methyl-6-(3-(trifluoromethoxy)phenyl)pyridin-3-ylamino)benzoate;
2-((5-Cyclopropyl-6-(3-methoxyphenyl)pyridin-3-ylamino)-5-methylbenzoic acid;
Ethyl 2-(5-cyclopropyl-6-(3-methoxyphenyl)pyridin-3-ylamino)-5-methylbenzoate;
2-((6-(2-Fluoro-5-isopropoxyphenyl)pyridin-3-ylamino)-5-methylbenzoic acid;
2-((6-(3-Isopropoxyphenyl)-5-methylpyridin-3-ylamino)-5-methylbenzoic acid;
Ethyl 2-(6-(3-isopropoxyphenyl)-5-methylpyridin-3-ylamino)-5-methylbenzoate;
2-((6-(3-Cyclopropoxyphenyl)-5-methylpyridin-3-ylamino)-5-methylbenzoic acid;
*tert*-Butyl 2-(6-(3-cyclopropoxyphenyl)-5-methylpyridin-3-ylamino)-5-methylbenzoate;
2-((6-(2-Chlorophenyl)-5-methylpyridin-3-ylamino)-5-methylbenzoic acid;
*tert*-Butyl 2-(6-(2-chlorophenyl)-5-methylpyridin-3-ylamino)-5-methylbenzoate;
2-((6-(3-Carbamoylphenyl)-5-methylpyridin-3-ylamino)-5-methylbenzoic acid;
Ethyl 2-(6-(3-carbamoylphenyl)-5-methylpyridin-3-ylamino)-5-methylbenzoate;
2-((6-(2-Fluoro-5-methoxyphenyl)-4-methylpyridin-3-ylamino)-5-methylbenzoic acid;
Ethyl 2-(6-(2-fluoro-5-methoxyphenyl)-4-methylpyridin-3-ylamino)-5-methylbenzoate;
2-((6-(3-Methoxyphenyl)-5-(trifluoromethyl)pyridin-3-ylamino)-5-methylbenzoic acid;
Ethyl 2-(6-(3-methoxyphenyl)-5-(trifluoromethyl)pyridin-3-ylamino)-5-methylbenzoate;
2-((6-(3-(Dimethylcarbamoyl)phenyl)-5-methylpyridin-3-ylamino)-5-methylbenzoic acid;
Ethyl 2-(6-(3-(dimethylcarbamoyl)phenyl)-5-methylpyridin-3-ylamino)-5-methylbenzoate;
2-((6-(3-Isopropoxyphenyl)-5-methylpyridin-3-ylamino)-3-methylbenzoic acid;
*tert*-Butyl 2-(6-(3-isopropoxyphenyl)-5-methylpyridin-3-ylamino)-3-methylbenzoate;
3-Methyl-2-(5-methyl-6-phenylpyridin-3-ylamino)benzoic acid;
*tert*-Butyl 3-methyl-2-(5-methyl-6-phenylpyridin-3-ylamino)benzoate;
2-((6-(2-Chlorophenyl)pyridin-3-ylamino)-5-methylbenzoic acid;
*tert*-Butyl 2-(6-(2-chlorophenyl)pyridin-3-ylamino)-5-methylbenzoate;
3-Fluoro-2-(6-(3-methoxyphenyl)-5-methylpyridin-3-ylamino)benzoic acid;
*tert*-Butyl 3-fluoro-2-(6-(3-methoxyphenyl)-5-methylpyridin-3-ylamino) benzoate;
5-Cyclopropyl-2-(5-methyl-6-(3-(trifluoromethoxy)phenyl)pyridin-3-ylamino)benzoic acid;
Ethyl 5-cyclopropyl-2-(5-methyl-6-(3-(trifluoromethoxy)phenyl)pyridin-3-ylamino)benzoate;
5-Cyclopropyl-2-(5-methyl-6-phenylpyridin-3-ylamino)benzoic acid;
Ethyl 5-cyclopropyl-2-(5-methyl-6-phenylpyridin-3-ylamino)benzoate;
5-Methyl-2-(5-methyl-6-(2-(trifluoromethyl)phenyl)pyridin-3-ylamino)benzoic acid;
*tert*-Butyl 5-methyl-2-(5-methyl-6-(2-(trifluoromethyl)phenyl)pyridin-3-ylamino)benzoate;
2-((6-(3-Chlorophenyl)-5-methylpyridin-3-ylamino)-5-methylbenzoic acid;
*tert*-Butyl 2-(6-(3-chlorophenyl)-5-methylpyridin-3-ylamino)-5-methylbenzoate;
2-((6-(2-Fluorophenyl)-5-methylpyridin-3-ylamino)-5-methylbenzoic acid;
*tert*-Butyl 2-(6-(2-fluorophenyl)-5-methylpyridin-3-ylamino)-5-methylbenzoate;
5-Methyl-2-(5-methyl-6-(quinolin-5-yl)pyridin-3-ylamino)benzoic acid;
*tert*-Butyl 5-methyl-2-(5-methyl-6-(quinolin-5-yl)pyridin-3-ylamino)benzoate;
2-((3'-Fluoro-3-methyl-2,4'-bipyridin-5-ylamino)-5-methylbenzoic acid;
*tert*-Butyl 2-(3'-fluoro-3-methyl-2,4'-bipyridin-5-ylamino)-5-methylbenzoate;
5-Methyl-2-(5-methyl-6-(pyrazin-2-yl)pyridin-3-ylamino)benzoic acid;
*tert*-Butyl 5-methyl-2-(5-methyl-6-(pyrazin-2-yl)pyridin-3-ylamino)benzoate;
5-Cyclopropyl-2-(6-phenyl-5-(trifluoromethyl)pyridin-3-ylamino)benzoic acid;
Ethyl 5-cyclopropyl-2-(6-phenyl-5-(trifluoromethyl)pyridin-3-ylamino)benzoate;
5-Cyclopropyl-2-(6-(3-methoxyphenyl)-5-(trifluoromethyl)pyridin-3-ylamino)benzoic acid;
Ethyl 5-cyclopropyl-2-(6-(3-methoxyphenyl)-5-(trifluoromethyl)pyridin-3-ylamino)benzoate;
5-Chloro-2-(6-(2-fluorophenyl)pyridin-3-ylamino)benzoic acid;
5-Chloro-2-(6-(2-chlorophenyl)pyridin-3-ylamino)benzoic acid;
5-Chloro-2-(6-(quinolin-5-yl)pyridin-3-ylamino)benzoic acid;
2-((6-(2-Chlorophenyl)pyridin-3-ylamino)-5-cyclopropylbenzoic acid;
Ethyl 2-(6-(2-chlorophenyl)pyridin-3-ylamino)-5-cyclopropylbenzoate;
5-Chloro-2-(6-(2-(trifluoromethyl)phenyl)pyridin-3-ylamino)benzoic acid;
5-Fluoro-2-(6-(2-(trifluoromethyl)phenyl)pyridin-3-ylamino)benzoic acid;
2-((3'-Fluoro-2,4'-bipyridin-5-ylamino)-5-methylbenzoic acid;
2-((2-(2-Fluorophenyl)pyrimidin-5-ylamino)-5-methylbenzoic acid;
tert-Butyl 2-(2-(2-fluorophenyl)pyrimidin-5-ylamino)-5-methylbenzoate;
2-((6-(2,6-Difluorophenyl)pyridin-3-ylamino)-5-methylbenzoic acid;
Ethyl 2-(6-(2,6-difluorophenyl)pyridin-3-ylamino)-5-methylbenzoate;
2-((2-(2-Chlorophenyl)pyrimidin-5-ylamino)-5-cyclopropylbenzoic acid;
Methyl 2-(2-(2-chlorophenyl)pyrimidin-5-ylamino)-5-cyclopropylbenzoate;
2-((2-(2-Chlorophenyl)pyrimidin-5-ylamino)-5-methylbenzoic acid;
*tert*-Butyl 2-(2-(2-chlorophenyl)pyrimidin-5-ylamino)-5-methylbenzoate;
5-Methyl-2-(5-methyl-6-(3-(pyrrolidine-1-carbonyl)phenyl)pyridin-3-ylamino)benzoic acid;
2-((6-(3-(Cyclopropylcarbamoyl)phenyl)-5-methylpyridin-3-ylamino)-5-methylbenzoic acid;
5-Cyclopropyl-2-(2-(2-fluorophenyl)pyrimidin-5-ylamino)benzoic acid;
2-((2-(2-trifluoromethylphenyl)pyrimidin-5-ylamino)-5-cyclopropylbenzoic acid;
2-((2-*o*-tolylpyrimidin-5-ylamino)-5-cyclopropylbenzoic acid;
2-((2-(2-cyclopropoxyphenyl)pyrimidin-5-ylamino)-5-cyclopropylbenzoic acid;
2-((2-(2,5-difluorophenyl)pyrimidin-5-ylamino)-5-cyclopropylbenzoic acid;
2-((2-(2,3-difluorophenyl)pyrimidin-5-ylamino)-5-cyclopropylbenzoic acid;
2-((2-(2-fluoro-5-chlorophenyl)pyrimidin-5-ylamino)-5-cyclopropylbenzoic acid;
2-((2-(2-trifluoromethylphenyl)pyrimidin-5-ylamino)-5-methylbenzoic acid;
2-((2-(2-fluoro-5-trifluoromethoxyphenyl)pyrimidin-5-ylamino)-5-cyclopropylbenzoic acid;
2-((6-(2-trifluoromethylphenyl)pyridin-3-ylamino)-5-methylbenzoic acid;
2-((6-phenylpyridin-3-ylamino)-5-cyclopropylbenzoic acid;
2-((6-(2-fluorophenyl)pyridin-3-ylamino)-5-cyclopropylbenzoic acid;
2-((6-(3,5-difluoropyridin-4-yl)pyridin-3-ylamino)-5-methylbenzoic acid;
2-((6-(3-cyclopropylcarbamoylphenyl)pyridin-3-ylamino)-5-methylbenzoic acid;
2-((6-(2,4-difluorophenyl)pyridin-3-ylamino)-5-methylbenzoic acid;
2-((6-(2,5-difluorophenyl)pyridin-3-ylamino)-5-methylbenzoic acid;
2-((6-(2-fluorophenyl)pyridin-3-ylamino)-5-cyclopropyl-3-fluorobenzoic acid;
2-((6-(2,3,6-trifluorophenyl)pyridin-3-ylamino)-5-methylbenzoic acid;
2-((6-(3-(5-methyl-1,3,4-oxadiazol-2-yl)phenyl)pyridin-3-ylamino)-5-methylbenzoic acid;
2-((5-methyl-6-(pyrimidin-5-yl)pyridin-3-ylamino)-5-methylbenzoic acid;
2-((6-(2,3-difluorophenyl)pyridin-3-ylamino)-5-methylbenzoic acid;
2-((6-(5-fluoro-2-methoxyphenyl)-5-methylpyridin-3-ylamino)-5-methylbenzoic acid;
2-((6-(4-carbamoylphenyl)-5-methylpyridin-3-ylamino)-5-methylbenzoic acid
or a pharmaceutically acceptable salt or N-oxide thereof;

8. A combination for use according to claim 1 wherein the DHODH inhibitor is 5-Methyl-2-(6-(3-(trifluoromethyl)phenyl)pyridin-3-ylamino)benzoic acid; 5-cyclopropyl-2-(2-(2,6-difluorophenyl)pyrimidin-5-ylamino)benzoic acid; 2-(6-(2,6-difluorophenyl)pyridin-3-ylamino)-5-methylbenzoic acid; 2-(6-(3-(cyclopropylcarbamoyl)phenyl)-5-methylpyridin-3-ylamino)-5-methylbenzoic acid
or a pharmaceutically acceptable salt or N-oxide thereof.

9. A combination for use according to claim 8 wherein the DHODH inhibitor is 5-cyclopropyl-2-(2-(2,6-difluorophenyl)pyrimidin-5-ylamino)benzoic acid or a pharmaceutically acceptable salt or N-oxide thereof.

10. A combination for use according to any one of the preceding claims wherein:
(1) the active ingredients (a) and (b) form part of a single pharmaceutical composition; and/or
(2) the combination further comprises (c) another compound selected from:
(i) Anti-TNF-alpha monoclonal antibodies such as Infliximab, Certolizumab pegol, Golimumab, Adalimumab and AME-527 from Applied Molecular Evolution;
(ii) TNF-alpha Antagonists such as Etanercept, Lenercept, Onercept and Pegsunercept;
(iii) Calcineurin (PP-2B) Inhibitors / INS Expression Inhibitors such as cyclosporine A, Tacrolimus and ISA-247 from Isotechnika;
(iv) IL-1 Receptor Antagonists such as Anakinra and AMG-719 from Amgen;
(v) Anti-CD20 monoclonal antibodies such as Rituximab, Ofatumumab, Ocrelizumab and TRU-015 from Trubion Pharmaceuticals;
(vi) p38 Inhibitors such as AMG-548 (from Amgen), ARRY-797 (from Array Biopharma), Chlormethiazole edisylate, Doramapimod, PS-540446 (from BMS), SB-203580, SB-242235, SB-235699, SB-281832, SB-681323, SB-856553 (all from GlaxoSmithKline), KC-706 (from Kemia), LEO-1606, LEO-15520 (all from Leo), SC-80036, SD-06 (all from Pfizer), RWJ-67657 (from R.W. Johnson), RO-3201195, RO-4402257 (all from Roche), AVE-9940 (from Aventis), SCIO-323, SCIO-469 (all from Scios), TA-5493 (from Tanabe Seiyaku), and VX-745 and VX-702 (all from Vertex);
(vii) NF-kappaB (NFKB) Activation Inhibitors such as Sulfasalazine and Iguratimod;
(viii) Another dihydrofolate reductase (DHFR) inhibitor such as Aminopterin and CH-1504 from Chelsea;
(ix) Janus kinase (JAK) inhibitors, such as CP-690, 550 from Pfizer and INCB-18424, from Incyte;
(x) MEK inhibitor, such as ARRY-162 from Array;
(xi) Sphingosine-1 phosphate receptor agonists, such as fingolimod (Novartis);
(xii) Interferons comprising Interferon beta 1a such as Avonex from Biogen Idec, CinnoVex from CinnaGen and Rebif from Merck Serono, and Interferon beta 1b such as Betaferon from Schering and Betaseron from Berlex;
(xiii) Inmunomodulators suchs as BG-12 (fumaric acid derivative) from Biogen Idec/Fumapharm AG; laquinimod (Teva and Active Biotech) or glatiramer acetate (Teva); and
(xiv) Adenosine aminohydrolase inhibitors such as Cladribine from Merck Serono.

11. A product comprising (a) methotrexate and (b) a DHODH inhibitor as defined in any one of claims 1 to 9, as a combined preparation for simultaneous, separate or sequential use in the treatment of a human or animal patient suffering from or susceptible to a pathological condition or disease as defined in claim 1,
wherein
(i) the methotrexate (a) is for administration at a dosage regime which involves administration of 0.015 to 3 mg/kg/week of methotrexate and the DHODH inhibitor (b) is for administration at a dosage regime which involves administration of 0.03 to 30 mg/kg/day of DHODH inhibitor; or
(ii) the combination is for use in treating a human or animal patient suffering from hepatic impairment, liver fibrosis, hepatitis, cirrhosis or liver cancer,
which product optionally further comprises an active compound (c), as defined in claim 10.

12. A kit of parts comprising (b) a DHODH inhibitor as defined in any one of claims 1 to 9 together with instructions for simultaneous, separate or sequential use in combination with (a) methotrexate for the treatment of a human or animal patient suffering from or susceptible to a pathological condition or disease as defined in claim 1,
wherein
(i) the methotrexate (a) is for administration at a dosage regime which involves administration of 0.015 to 3 mg/kg/week of methotrexate and the DHODH inhibitor (b) is for administration at a dosage regime which involves administration of 0.03 to 30 mg/kg/day of DHODH inhibitor; or
(ii) the combination is for use in treating a human or animal patient suffering from hepatic impairment, liver fibrosis, hepatitis, cirrhosis or liver cancer,
which kit optionally further comprises an active compound (c), as defined in claim 10.

13. A package comprising (b) a DHODH inhibitor as defined in any one of claims 1 to 9 and (a) methotrexate, for simultaneous, separate or sequential use in the treatment of a pathological condition or disease as defined in claim 1,
wherein
(i) the methotrexate (a) is for administration at a dosage regime which involves administration of 0.015 to 3 mg/kg/week of methotrexate and the DHODH inhibitor (b) is for administration at a dosage regime which involves administration of 0.03 to 30 mg/kg/day of DHODH inhibitor; or
(ii) the combination is for use in treating a human or animal patient suffering from hepatic impairment, liver fibrosis, hepatitis, cirrhosis or liver cancer,
which package optionally further comprises an active compound (c), as defined in claim 10.

14. A DHODH inhibitor as defined in any one of claims 1 to 9 for use in treating a human or animal patient suffering from or susceptible to a pathological condition or disease as defined in claim 1, wherein the human or animal patient is suffering from hepatic impairment, liver fibrosis, cirrhosis or liver cancer.

15. A combination comprising (a) methotrexate and (b) a DHODH inhibitor as defined in claim 8 or 9.

## Patentansprüche

1. Kombination für simultane, separate oder sequentielle Verwendung für die Behandlung eines pathologischen Zustands oder einer Krankheit, welche durch Inhibierung von Dihydroorotatdehydrogenase gelindert werden kann, welche ausgewählt ist aus rheumatischer Arthritis, Psoriasisarthritis, ankylosierender Spondilytis, multipler Sklerose, Wegener'scher Granulomatose, systemischem Lupus erythematodes, Psoriasis und Sarkoidose, umfassend (a) Methotrexat sowie (b) einen nichthepatotoxischen DHODH-Inhibitor der Formel (I): 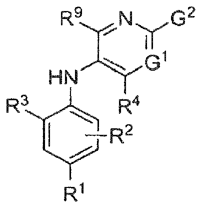 wobei:
R¹ ausgewählt ist aus der Gruppe, bestehend aus Wasserstoffatomen, Halogenatomen, C₁₋₄-Alkyl, C₃₋₄-Cycloalkyl, -CF₃ und -OCF₃,
R² ausgewählt ist aus der Gruppe, bestehend aus Wasserstoffatomen, Halogenatomen und C₁₋₄-Alkylgruppen,
R³ ausgewählt ist aus der Gruppe, bestehend aus -COOR⁵-, -CONHR⁵-, Tetrazolyl-, -SO₂NHR⁵- und -CONHSO₂R⁵-Gruppen, wobei R⁵ ausgewählt ist aus der Gruppe, bestehend aus einem Wasserstoffatom und linearen oder verzweigten C₁₋₄-Alkylgruppen,
R⁴ ausgewählt ist aus der Gruppe, bestehend aus einem Wasserstoffatom und einer C₁₋₄-Alkylgruppe,
R⁹ ausgewählt ist aus der Gruppe, bestehend aus einem Wasserstoffatom und einer Phenylgruppe,
G¹ eine Gruppe darstellt, welche ausgewählt ist aus N und CR⁶, wobei R⁶ ausgewählt ist aus der Gruppe, bestehend aus Wasserstoffatomen, Halogenatomen, C₁₋₄-Alkyl-, C₃₋₄-Cycloalkyl-, C₁₋₄-Alkoxy-, -CF₃-, -OCF₃-, monocyclischen N-haltigen C₅₋₇-Heteroaryl-, monocyclischen N-haltigen C₃₋₇-Heterocyclylgruppen und C₆₋₁₀-Arylgruppen, wobei die C₆₋₁₀-Arylgruppen optional substituiert sind mit einem oder mehreren Substituenten, ausgewählt aus Halogenatomen und C₁₋₄-Alkylgruppen,
G² eine Gruppe darstellt, gewählt aus:
• einem Wasserstoffatom, einer Hydroxylgruppe, einem Halogenatom, einer C₃₋₄-Cycloalkylgruppe, einer C₁₋₄-Alkoxygruppe und -NR^{a}R^{b}, wobei
R^{a} eine C₁₋₄-Alkylgruppe darstellt und R^{b} ausgewählt ist aus einer Gruppe, bestehend aus einer C₁₋₄-Alkylgruppe und einer C₁₋₄-Alkoxy-C₁₋₄-alkylgruppe, oder
R^{a} und R^{b} gemeinsam mit dem Stickstoffatom, an das sie gebunden sind, einen gesättigten 6- bis 8-gliedrigen heterocyclischen Ring bilden, welcher optional ein Sauerstoffatom als zusätzliches Heteroatom enthält,
• einem monocyclischen oder bicyclischen 5- bis 10-gliedrigen heteroaromatischen Ring, welcher ein oder mehrere Stickstoffatome enthält, welcher optional substituiert ist mit einem oder mehreren Substituenten, ausgewählt aus Halogenatomen, C₁₋₄-Alkyl, C₁₋₄-Alkoxy, C₃₋₄-Cycloalkyl, C₃₋₄-Cycloalkoxy, -CF₃, -OCF₃ und -CONR⁷R⁸, wobei R⁷ und R⁸ unabhängig voneinander ausgewählt sind aus Wasserstoffatom, linearen oder verzweigten C₁₋₄-Alkylgruppen, C₃₋₇-Cycloalkylgruppen, oder R⁷ und R⁸ gemeinsam mit dem Stickstoffatom, an das sie gebunden sind, eine Gruppe bilden mit der Formel 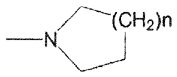 wobei n eine ganze Zahl von 0 bis 3 ist,
und
• einer Phenylgruppe, welche optional substituiert ist mit einem oder mehreren Substituenten, ausgewählt aus Halogenatomen, C₁₋₄-Alkyl, Hydroxyl, C₁₋₄-Alkoxy, C₃₋₄-Cycloalkyl, C₃₋₄-Cycloalkoxy, Cyano, -CF₃, -OCF₃, -CONR⁷R⁸, Oxadiazolyl-, Triazolyl-, Pyrazolyl- und Imidazolylgruppen, wobei die Oxadiazolyl-, Triazolyl-, Pyrazolyl- und Imidazolylgruppen optional substituiert sind mit C₁₋₄-Alkyl oder C₃₋₇-Cycloalkylgruppen, und wobei R⁷ und R⁸ unabhängig ausgewählt sind aus Wasserstoffatom, linearen oder verzweigten C₁₋₄-Alkylgruppen, C₃₋₇-Cycloalkylgruppen, oder R⁷ und R⁸ gemeinsam mit dem Stickstoffatom, an das sie gebunden sind, eine Gruppe bilden der Formel 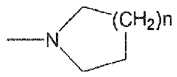 wobei n eine ganze Zahl von 0 bis 3 ist
oder, wenn G¹ für CR⁶ steht, G² gemeinsam mit R⁶ eine nichtaromatische carbocyclische C₅₋₁₀-Gruppe oder eine C₆₋₁₀-Arylgruppe bildet,
oder pharmazeutisch verträgliche Salze oder N-Oxid davon,
wobei
(i) das Methotrexat (a) für die Verabreichung in einem Dosierungs-Regime vorgesehen ist, welches die Verabreichung von 0,015 bis 3 mg/kg/Woche Methotrexat beinhaltet und der DHODH-Inhibitor (b) für die Verabreichung in einem Dosierungs-Regime vorgesehen ist, welches die Verabreichung von 0,03 bis 30 mg/kg/Tag des DHODH-Inhibitors beinhaltet; oder
(ii) die Kombination für die Verwendung bei der Behandlung eines menschlichen oder tierischen Patienten bestimmt ist, welcher an einer Leberschädigung, Leberfibrose, Hepatitis, Zirrhose oder Leberkrebs leidet.

2. Kombination zur Verwendung gemäß Anspruch 1, wobei:
R¹ ausgewählt ist aus der Gruppe, bestehend aus Wasserstoffatomen, Fluoratomen, Chloratomen, Bromatomen, C₁₋₄-Alkyl-, C₃₋₄-Cycloalkyl- und -CF₃-Gruppen;
R² ausgewählt ist aus der Gruppe, bestehend aus Wasserstoffatomen, Halogenatomen und einer Methylgruppe;
R³ ausgewählt ist aus der Gruppe, bestehend aus -COOR⁵-, -CONHR⁵-, Tetrazolyl-, -SO₂NHR⁵- und -CONHSO₂R⁵-Gruppen, wobei R⁵ ausgewählt ist aus der Gruppe, bestehend aus einem Wasserstoffatom und linearen oder verzweigten C₁₋₄-Alkylgruppen,
R⁴ ausgewählt ist aus der Gruppe, bestehend aus einem Wasserstoffatom und einer C₁₋₄-Alkylgruppe,
R⁹ ausgewählt ist aus der Gruppe, bestehend aus einem Wasserstoffatom und einer Phenylgruppe,
G¹ ausgewählt ist aus der Gruppe, bestehend aus Stickstoffatomen, CCl-, CF-, CH-, C(CH₃)-, C(Cyclopropyl)-, C(Phenyl)- und C(CF₃)-Gruppen; und/oder
G² eine Gruppe darstellt, welche ausgewählt ist aus:
• einem Wasserstoffatom, einem Halogenatom, einer C₃₋₄-Cycloalkylgruppe, einer C₁₋₂-Alkoxygruppe und -NR^{a}R^{b}, wobei
R^{a} eine C₁₋₂-Alkylgruppe darstellt und R^{b} ausgewählt ist aus der Gruppe, bestehend aus C₁₋₂-Alkylgruppen und C₁₋₂-Alkoxy-C₁₋₂-alkylgruppen, oder
R^{a} und R^{b} gemeinsam mit dem Stickstoffatom, an das sie gebunden sind, einen gesättigten 6- oder 7-gliedrigen heterocyclischen Ring bilden, welcher optional ein Sauerstoffatom als zusätzliches Heteroatom enthält,
• einem monocyclischen oder bicyclischen 5- bis 10-gliedrigen heteroaromatischen Ring mit einem oder zwei Stickstoffatomen, welcher optional mit einem oder mehreren Substituenten substituiert ist, ausgewählt aus Halogenatomen und C₁₋₄-Alkylgruppen,
und
• einer Phenylgruppe, welche optional mit einem, zwei oder drei Substituenten substituiert ist, ausgewählt aus Halogenatomen, C₁₋₄-Alkyl-, Hydroxyl-, C₁₋₄-Alkoxy-, C₃₋₄-Cycloalkyl-, C₃₋₄-Cycloalkoxy-, Cyano-, -CF₃-, -OCF₃-, -CONR⁷R⁸- und Oxadiazolylgruppen, wobei die Oxadiazolylgruppen optional substituiert sind mit einer C₁₋₄-Alkyl- oder einer C₃₋₇-Cycloalkylgruppe und wobei R⁷ und R⁸ unabhängig voneinander gewählt sind aus Wasserstoffatomen, linearen oder verzweigten C₁₋₄-Alkylgruppen, C₃₋₄-Cycloalkylgruppen, oder R⁷ und R⁸ gemeinsam mit dem Stickstoffatom, an das sie gebunden sind, eine Gruppe bilden der Formel 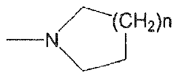 wobei n 1 oder 2 ist,
oder G¹ und G² gemeinsam eine nichtaromatische carbocyclische C₆-Gruppe oder eine Phenylgruppe bilden;
oder ein pharmazeutisch verträgliches Salz oder N-Oxid davon.

3. Kombination zur Verwendung gemäß einem der Ansprüche 1 oder 2, wobei
(i) G² eine Gruppe darstellt, ausgewählt aus:
• einem Wasserstoffatom, Fluoratom, einer Cyclopropylgruppe, Methoxygruppe, -NMeEt, -NEt₂, -N(Me)-(CH₂)₂-O-CH₃, 6-Morpholinyl, Azepan-1-yl und Piperidin-1-yl,
• einem Pyridinyl-, Pyrimidinyl-, Chinolinyl- oder Pyrazinylring, optional substituiert mit einem oder zwei Substituenten, ausgewählt aus Me und F
und
• einer Phenylgruppe, welche optional substituiert ist mit einem, zwei oder drei Substituenten, ausgewählt aus Fluor-, Chlor-, Methyl-, Hydroxyl-, Methoxy-, Ethoxy-, Isopropyloxy-, Cyclopropyl-, Cyclopropyloxy-, Cyano-, -CF₃-, -OCF₃-, Oxadiazolyl- und -CONR⁷R⁸-Gruppen, wobei die Oxadiazolylgruppen optional mit einer Methylgruppe substituiert sind und wobei R⁷ und R⁸ unabhängig ausgewählt sind aus Wasserstoffatom, Methylgruppe, Isopropylgruppe, Cyclopropylgruppe, oder R⁷ und R⁸ gemeinsam mit dem Stickstoffatom, an das sie gebunden sind, eine Gruppe bilden der Formel 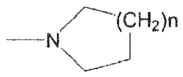 wobei n 1 ist,
oder G¹ und G² miteinander eine nichtaromatische carbocyclische C₆-Gruppe oder eine Phenylgruppe bilden; und/oder
(ii) G² eine Gruppe darstellt, ausgewählt aus Methoxygruppe, Cyclopropylgruppe und optional substituierten Phenyl-, Pyridyl-, Chinolinyl-, Pyrimidinyl- und Pyrazinylgruppen.

4. Kombination zur Verwendung gemäß einem der Ansprüche 1 bis 3, wobei:
(i) R¹ gewählt ist aus der Gruppe, bestehend aus C₁₋₄-Alkyl-, C₃₋₄-Cycloalkyl- und -CF₃-Gruppen, typischerweise gewählt aus der Gruppe, bestehend aus Methyl- und Cyclopropylgruppen, vorzugsweise eine Cyclopropylgruppe ist; und/oder
(ii) R² gewählt ist aus einem Wasserstoff- oder Halogenatom, typischerweise ein Wasserstoffatom ist; und/oder
(iii) R³ gewählt ist aus der Gruppe, bestehend aus COOR⁵-, -CONHR⁵- und Tetrazolylgruppen, typischerweise eine -COOH-Gruppe ist; und/oder
(iv) R⁴ ein Wasserstoffatom oder eine Methylgruppe darstellt, typischerweise ein Wasserstoffatom; und/oder
(v) R⁹ ein Wasserstoffatom darstellt; und/oder
(vi) G¹ gewählt ist aus der Gruppe, bestehend aus Stickstoffatomen und CH-, C (CH₃) -, C (Cyclopropyl) -, C (Phenyl) - und C (CF₃) -Gruppen; und/oder
(vii) G² eine Gruppe darstellt, ausgewählt aus optional substituierten Phenyl-, Pyridyl-, Chinolinyl-, Pyrimidinyl- und Pyrazinylgruppen, typischerweise gewählt aus der Gruppe, bestehend aus einer gegebenenfalls substituierten Phenyl-, 4-Pyridyl-, 5-Chinolynyl- und 2-Pyrazinylgruppe.

5. Kombination zur Verwendung gemäß Anspruch 1 oder 2, wobei R¹ gewählt ist aus einer Methyl- oder Cyclopropylgruppe, R² ein Wasserstoffatom darstellt, R³ eine COOH-Gruppe ist, R⁴ ein Wasserstoffatom oder eine Methylgruppe darstellt, G¹ ausgewählt ist aus Stickstoffatomen und CH-, C(CH₃)-, C (Cyclopropyl) -, C (Phenyl) - und C (CF₃) -Gruppe und G² eine Gruppe darstellt, ausgewählt aus der Gruppe, bestehend aus einer optional substituierten Phenyl-, 4-Pyridyl-, 5-Chinolynyl- und 2-Pyrazinylgruppe; und wobei R⁹ optional ein Wasserstoffatom darstellt.

6. Kombination zur Verwendung gemäß Anspruch 1 oder 2, wobei R¹ ausgewählt ist aus einer Methyl- oder Cyclopropylgruppe, R² ein Wasserstoffatom darstellt, R³ eine COOH-Gruppe ist, R⁴ ein Wasserstoffatom darstellt, G¹ ausgewählt ist aus Stickstoffatomen und CH-, C (CH₃) - und C(CF₃)-Gruppen und G² eine Phenylgruppe darstellt, die optional mit einem oder zwei Substituenten substituiert ist, ausgewählt aus Chlor, Fluor, Methoxy, Ethoxy, Isopropoxy, Trifluormethoxy und -CONR⁷R⁸, wobei R⁷ Wasserstoff ist und R⁸ Cyclopropyl ist oder R⁷ und R⁸, gemeinsam mit dem Stickstoffatom, an das sie gebunden sind, eine Gruppe bilden der Formel 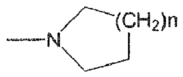 wobei n 1 ist.

7. Kombination zur Verwendung gemäß Anspruch 1, wobei der DHODH-Inhibitor einer ist aus:
5-Cyclopropyl-2-(2-phenylpyrimidin-5-ylamino)benzoesäure;
2-((6-Cyclopropyl-5-methylpyridin-3-ylamino)-5-methylbenzoesäure;
5-((2-Carboxy-4-CyClopropylphenylamino)-3-methyl-2-phenylpyridin-1-oxid;
5-Methyl-2-(6-(3-(trifluormethyl)phenyl)pyridin-3-ylamino)benzoesäure;
5-Cyclopropyl-2-(2-(2,6-difluor-4-hydroxyphenyl)pyrimidin-5-ylamino)benzoesäure;
5-Cyclopropyl-2-(6-methoxy-5-phenylpyridin-3-ylamino)benzoesäure;
2-((5-Fluor-6-phenylpyridin-3-ylamino)-5-methylbenzoesäure;
2-((6-(Ethyl(methyl)amino)-5-methylpyridin-3-ylamino)-5-methylbenzoesäure;
5-Cyclopropyl-2-(3'-fluor-2,4'-bipyridin-5-ylamino)benzoesäure;
2-((6-(Diethylamino)-5-methylpyridin-3-ylamino)-5-methylbenzoesäure;
2-((6-((2-Methoxyethyl)(methyl)amino)-5-methylpyridin-3-ylamino)-5-methylbenzoesäure;
2-((5-Chlor-6-phenylpyridin-3-ylamino)-5-methylbenzoesäure;
5-Cyclopropyl-2-(2-(2-CyClopropylphenyl)pyrimidin-5-ylamino)benzoesäure;
5-Cyclopropyl-2-(5-phenylpyridin-3-ylamino)benzoesäure;
5-Methyl-2-(chinolin-3-ylamino)benzoesäure;
5-Methyl-2-(5,6,7,8-tetrahydrochinolin-3-ylamino)benzoesäure;
2-((5-Chlor-2-phenylpyridin-3-ylamino)-5-methylbenzoesäure;
5-Cyclopropyl-2-(5,6-diphenylpyridin-3-ylamino)benzoesäure;
5-Cyclopropyl-2-(2-(2,6-difluorphenyl)pyrimidin-5-ylamino)benzoesäure;
5-Cyclopropyl-2-(5-methylpyridin-3-ylamino)benzoesäure;
2-((2-(3-Cyclopropoxyphenyl)pyrimidin-5-ylamino)-5-cyclopropylbenzoesäure;
5-Cyclopropyl-2-(6-cyclopropyl-5-phenylpyridin-3-ylamino)benzoesäure;
2-((6-(2-Cyclopropylphenyl)-5-methylpyridin-3-ylamino)-5-methylbenzoesäure;
2-((6-(2-Cyanophenyl)-5-methylpyridin-3-ylamino)-5-methylbenzoesäure;
2-((2-(3-Chlorphenyl)pyrimidin-5-ylamino)-5-cyclopropylbenzoesäure;
5-Methyl-2-(6-phenyl-5-(trifluormethyl)pyridin-3-ylamino)benzoesäure;
2-((6-(3-Methoxyphenyl)-5-phenylpyridin-3-ylamino)-5-methylbenzoesäure;
2-((2,3'-Bipyridin-5-ylamino)-5-cyclopropylbenzoesäure;
2-((3'-Chlor-2,4'-bipyridin-5-ylamino)-5-methylbenzoesäure;
5-Methyl-2-(3-methyl-2,2'-bipyridin-5-ylamino)benzoesäure;
2-((5,6-Difluorpyridin-3-ylamino)-5-methylbenzoesäure;
2-((6-(3-Methoxyphenyl)pyridin-3-ylamino)benzoesäure;
2-((6-(3-Ethoxyphenyl)pyridin-3-ylamino)benzoesäure;
2-((6-(3-Ethoxyphenyl)pyridin-3-ylamino)-5-fluorbenzoesäure;
2-((6-(3-Ethoxyphenyl)-5-methylpyridin-3-ylamino)benzoesäure;
2-((6-(3-Ethoxyphenyl)pyridin-3-ylamino)-5-methylbenzoesäure;
2-((6-(3-Ethoxyphenyl)-5-methylpyridin-3-ylamino)-5-methylbenzoesäure;
2-((6-(3-Ethoxy-2-fluorphenyl)pyridin-3-ylamino)benzoesäure;
2-((6-(3-Ethoxyphenyl)-4-methylpyridin-3-ylamino)-5-methylbenzoesäure;
2-((6-(3-Ethoxyphenyl)-4-methylpyridin-3-ylamino)benzoesäure;
5-Brom-2-(6-(3-ethoxyphenyl)pyridin-3-ylamino)benzoesäure;
5-Chlor-2-(6-(3-ethoxyphenyl)pyridin-3-ylamino)benzoesäure;
2-((6-(5-Ethoxy-2-fluorphenyl)pyridin-3-ylamino)benzoesäure;
2-((6-(3-Ethoxyphenyl)-5-methylpyridin-3-ylamino)-5-(trifluormethyl)benzoesäure;
2-((6-(3-Methoxyphenyl)-5-methylpyridin-3-ylamino)-5-(trifluormethyl)benzoesäure;
2-((6-(3-Methoxyphenyl)-5-methylpyridin-3-ylamino)-5-methylbenzoesäure;
2-((6-(3-Methoxyphenyl)-5-methylpyridin-3-ylamino)-6-methylbenzoesäure;
5-Fluor-2-(6-(3-methoxyphenyl)-5-methylpyridin-3-ylamino)benzoesäure;
2-((6-(5-Ethoxy-2-fluorphenyl)pyridin-3-ylamino)-5-methylbenzoesäure;
2-((6-(2-Fluor-5-methoxyphenyl)-5-methylpyridin-3-ylamino)-5-methylbenzoesäure;
Ethyl-2-(6-(2-fluor-5-methoxyphenyl)-5-methylpyridin-3-ylamino)-5-methylbenzoat;
2-((6-(2-Fluorphenyl)pyridin-3-ylamino)-5-methylbenzoesäure;
2-((6-(3-Methoxyphenyl)-5-phenylpyridin-3-ylamino)-5-methylbenzoesäure;
Ethyl-2-(6-(3-methoxyphenyl)-5-phenylpyridin-3-ylamino)-5-methylbenzoat;
5-Methyl-2-(5-methyl-6-phenylpyridin-3-ylamino)benzoesäure;
Ethyl-5-methyl-2-(5-methyl-6-phenylpyridin-3-ylamino)benzoat;
5-Methyl-2-(5-methyl-6-(3-(trifluormethoxy)phenyl)pyridin-3-ylamino)benzoesäure;
Ethyl-5-methyl-2-(5-methyl-6-(3-(trifluormethoxy)phenyl)pyridin-3-ylamino)benzoat;
2-((5-Cyclopropyl-6-(3-methoxyphenyl)pyridin-3-ylamino)-5-methylbenzoesäure;
Ethyl-2-(5-cyclopropyl-6-(3-methoxyphenyl)pyridin-3-ylamino)-5-methylbenzoat;
2-((6-(2-Fluor-5-isopropoxyphenyl)pyridin-3-ylamino)-5-methylbenzoesäure;
2-((6-(3-Isopropoxyphenyl)-5-methylpyridin-3-ylamino)-5-methylbenzoesäure;
Ethyl-2-(6-(3-isopropoxyphenyl)-5-methylpyridin-3-ylamino)-5-methylbenzoat;
2-((6-(3-Cyclopropoxyphenyl)-5-methylpyridin-3-ylamino)-5-methylbenzoesäure;
tert-Butyl-2-(6-(3-cyclopropoxyphenyl)-5-methylpyridin-3-ylamino)-5-methylbenzoat;
2-((6-(2-Chlorphenyl)-5-methylpyridin-3-ylamino)-5-methylbenzoesäure;
tert-Butyl-2-(6-(2-chlorphenyl)-5-methylpyridin-3-ylamino)-5-methylbenzoat;
2-((6-(3-Carbamoylphenyl)-5-methylpyridin-3-ylamino)-5-methylbenzoesäure;
Ethyl-2-(6-(3-carbamoylphenyl)-5-methylpyridin-3-ylamino)-5-methylbenzoat;
2-((6-(2-Fluor-5-methoxyphenyl)-4-methylpyridin-3-ylamino)-5-methylbenzoesäure;
Ethyl-2-(6-(2-fluor-5-methoxyphenyl)-4-methylpyridin-3-ylamino)-5-methylbenzoat;
2-((6-(3-Methoxyphenyl)-5-(trifluormethyl)pyridin-3-ylamino)-5-methylbenzoesäure;
Ethyl-2-(6-(3-methoxyphenyl)-5-(trifluormethyl)pyridin-3-ylamino)-5-methylbenzoat;
2-((6-(3-(Dimethylcarbamoyl)phenyl)-5-methylpyridin-3-ylamino)-5-methylbenzoesäure;
Ethyl-2-(6-(3-(dimethylcarbamoyl)phenyl)-5-methylpyridin-3-ylamino)-5-methylbenzoat;
2-((6-(3-Isopropoxyphenyl)-5-methylpyridin-3-ylamino)-3-methylbenzoesäure;
tert-Butyl-2-(6-(3-isopropoxyphenyl)-5-methylpyridin-3-ylamino)-3-methylbenzoat;
3-Methyl-2-(5-methyl-6-phenylpyridin-3-ylamino)benzoesäure;
*tert*-Butyl-3-methyl-2-(5-methyl-6-phenylpyridin-3-ylamino)benzoat;
2-((6-(2-Chlorphenyl)pyridin-3-ylamino)-5-methylbenzoesäure;
*tert*-Butyl-2-(6-(2-chlorphenyl)pyridin-3-ylamino)-5-methylbenzoat;
3-Fluor-2-(6-(3-methoxyphenyl)-5-methylpyridin-3-ylamino)benzoesäure;
*tert*-Butyl-3-fluor-2-(6-(3-methoxyphenyl)-5-methylpyridin-3-ylamino)benzoat;
5-Cyclopropyl-2-(5-methyl-6-(3-(trifluormethoxy)phenyl)pyridin-3-ylamino)benzoesäure;
Ethyl-5-cyclopropyl-2-(5-methyl-6-(3-(trifluormethoxy)phenyl)pyridin-3-ylamino)benzoat;
5-Cyclopropyl-2-(5-methyl-6-phenylpyridin-3-ylamino)benzoesäure;
Ethyl-5-cyclopropyl-2-(5-methyl-6-phenylpyridin-3-ylamino)benzoat;
5-Methyl-2-(5-methyl-6-(2-(trifluormethyl)phenyl)pyridin-3-ylamino)benzoesäure;
*tert*-Butyl-5-methyl-2-(5-methyl-6-(2-(trifluormethyl)phenyl)pyridin-3-ylamino)benzoat;
2-((6-(3-Chlorphenyl)-5-methylpyridin-3-ylamino)-5-methylbenzoesäure;
*tert*-Butyl-2-(6-(3-chlorphenyl)-5-methylpyridin-3-ylamino)-5-methylbenzoat;
2-((6-(2-Fluorphenyl)-5-methylpyridin-3-ylamino)-5-methylbenzoesäure;
*tert*-Butyl-2-(6-(2-fluorphenyl)-5-methylpyridin-3-ylamino)-5-methylbenzoat;
5-Methyl-2-(5-methyl-6-(chinolin-5-yl)pyridin-3-ylamino)benzoesäure;
tert-Butyl-5-methyl-2-(5-methyl-6-(chinolin-5-yl)pyridin-3-ylamino)benzoat;
2-((3'-Fluor-3-methyl-2,4'-bipyridin-5-ylamino)-5-methylbenzoesäure;
tert-Butyl-2-(3'-fluor-3-methyl-2,4'-bipyridin-5-ylamino)-5-methylbenzoat;
5-Methyl-2-(5-methyl-6-(pyrazin-2-yl)pyridin-3-ylamino)benzoesäure;
tert-Butyl-5-methyl-2-(5-methyl-6-(pyrazin-2-yl)pyridin-3-ylamino)benzoat;
5-Cyclopropyl-2-(6-phenyl-5-(trifluormethyl)pyridin-3-ylamino)benzoesäure;
Ethyl-5-cyclopropyl-2-(6-phenyl-5-(trifluormethyl)pyridin-3-ylamino)benzoat;
5-Cyclopropyl-2-(6-(3-methoxyphenyl)-5-(trifluormethyl)pyridin-3-ylamino)benzoesäure;
Ethyl-5-cyclopropyl-2-(6-(3-methoxyphenyl)-5-(trifluormethyl)pyridin-3-ylamino)benzoat;
5-Chlor-2-(6-(2-fluorphenyl)pyridin-3-ylamino)benzoesäure;
5-Chlor-2-(6-(2-chlorphenyl)pyridin-3-ylamino)benzoesäure;
5-Chlor-2-(6-(chinolin-5-yl)pyridin-3-ylamino)benzoesäure;
2-((6-(2-Chlorphenyl)pyridin-3-ylamino)-5-cyclopropylbenzoesäure;
Ethyl-2-(6-(2-chlorphenyl)pyridin-3-ylamino)-5-cyclopropylbenzoat;
5-Chlor-2-(6-(2-(trifluormethyl)phenyl)pyridin-3-ylamino)benzoesäure;
5-Fluor-2-(6-(2-(trifluormethyl)phenyl)pyridin-3-ylamino)benzoesäure;
2-((3'-Fluor-2,4'-bipyridin-5-ylamino)-5-methylbenzoesäure;
2-((2-(2-Fluorphenyl)pyrimidin-5-ylamino)-5-methylbenzoesäure;
tert-Butyl-2-(2-(2-fluorphenyl)pyrimidin-5-ylamino)-5-methylbenzoat;
2-((6-(2,6-Difluorphenyl)pyridin-3-ylamino)-5-methylbenzoesäure;
Ethyl-2-(6-(2,6-difluorphenyl)pyridin-3-ylamino)-5-methylbenzoat;
2-((2-(2-Chlorphenyl)pyrimidin-5-ylamino)-5-cyclopropylbenzoesäure;
Methyl-2-(2-(2-chlorphenyl)pyrimidin-5-ylamino)-5-cyclopropylbenzoat;
2-((2-(2-Chlorphenyl)pyrimidin-5-ylamino)-5-methylbenzoesäure;
tert-Butyl-2-(2-(2-chlorphenyl)pyrimidin-5-ylamino)-5-methylbenzoat;
5-Methyl-2-(5-methyl-6-(3-(pyrrolidin-1-carbonyl)phenyl)pyridin-3-ylamino)benzoesäure;
2-((6-(3-(Cyclopropylcarbamoyl)phenyl)-5-methylpyridin-3-ylamino)-5-methylbenzoesäure;
5-Cyclopropyl-2-(2-(2-fluorphenyl)pyrimidin-5-ylamino)benzoesäure;
2-((2-(2-Trifluormethylphenyl)pyrimidin-5-ylamino)-5-cyclopropylbenzoesäure;
2-((2-o-Tolylpyrimidin-5-ylamino)-5-CyClopropylbenzoesäure;
2-((2-(2-Cyclopropoxyphenyl)pyrimidin-5-ylamino)-5-cyclopropylbenzoesäure;
2-((2-(2,5-Difluorphenyl)pyrimidin-5-ylamino)-5-cyclopropylbenzoesäure;
2-((2-(2,3-Difluorphenyl)pyrimidin-5-ylamino)-5-cyclopropylbenzoesäure;
2-((2-(2-Fluor-5-chlorphenyl)pyrimidin-5-ylamino)-5-cyclopropylbenzoesäure;
2-((2-(2-Trifluormethylphenyl)pyrimidin-5-ylamino)-5-methylbenzoesäure;
2-((2-(2-Fluor-5-trifluormethoxyphenyl)pyrimidin-5-ylamino)-5-cyclopropylbenzoesäure;
2-((6-(2-Trifluormethylphenyl)pyridin-3-ylamino)-5-methylbenzoesäure;
2-((6-Phenylpyridin-3-ylamino)-5-CyClopropylbenzoesäure;
2-((6-(2-Fluorphenyl)pyridin-3-ylamino)-5-cyclopropylbenzoesäure;
2-((6-(3,5-Difluorpyridin-4-yl)pyridin-3-ylamino)-5-methylbenzoesäure;
2-((6-(3-Cyclopropylcarbamoylphenyl)pyridin-3-ylamino)-5-methylbenzoesäure;
2-((6-(2,4-Difluorphenyl)pyridin-3-ylamino)-5-methylbenzoesäure;
2-((6-(2,5-Difluorphenyl)pyridin-3-ylamino)-5-methylbenzoesäure;
2-((6-(2-Fluorphenyl)pyridin-3-ylamino)-5-cyclopropyl-3-fluorbenzoesäure;
2-((6-(2,3,6-Trifluorphenyl)pyridin-3-ylamino)-5-methylbenzoesäure;
2-((6-(3-(5-Methyl-1,3,4-oxadiazol-2-yl)phenyl)pyridin-3-ylamino)-5-methylbenzoesäure;
2-((5-Methyl-6-(pyrimidin-5-yl)pyridin-3-ylamino)-5-methylbenzoesäure;
2-((6-(2,3-Difluorphenyl)pyridin-3-ylamino)-5-methylbenzoesäure;
2-((6-(5-Fluor-2-methoxyphenyl)-5-methylpyridin-3-ylamino)-5-methylbenzoesäure;
2-((6-(4-Carbamoylphenyl)-5-methylpyridin-3-ylamino)-5-methylbenzoesäure
oder ein pharmazeutisch verträgliches Salz oder N-Oxid davon.

8. Kombination zur Verwendung gemäß Anspruch 1, wobei der DHODH-Inhibitor 5-Methyl-2-(6-(3-(trifluormethyl)phenyl)pyridin-3-ylamino)benzoesäure; 5-Cyclopropyl-2-(2-(2,6-difluorphenyl)pyrimidin-5-ylamino)benzoesäure; 2-(6-(2,6-Difluorphenyl)pyridin-3-ylamino)-5-methylbenzoesäure; 2-(6-(3-(Cyclopropylcarbamoyl)phenyl)-5-methylpyridin-3-ylamino)-5-methylbenzoesäure
oder ein pharmazeutisch verträgliches Salz oder N-Oxid davon ist.

9. Kombination zur Verwendung gemäß Anspruch 8, wobei der DHODH-Inhibitor 5-Cyclopropyl-2-(2-(2,6-difluorphenyl)pyrimidin-5-ylamino)benzoesäure oder ein pharmazeutisch verträgliches Salz oder N-Oxid davon ist.

10. Kombination zur Verwendung gemäß einem der vorangehenden Ansprüche, wobei:
(1) die aktiven Bestandteile (a) und (b) einen Teil einer einzelnen pharmazeutischen Zusammensetzung bilden; und/oder
(2) die Kombination ferner (c) eine weitere Verbindung umfasst, ausgewählt aus:
(i) monoklonalen Anti-TNF-alpha-Antikörpern, wie zum Beispiel Infliximab, Certolizumab pegol, Golimumab, Adalimumab und AME-527 von Applied Molecular Evolution;
(ii) TNF-alpha-Antagonisten, wie zum Beispiel Etanercept, Lenercept, Onercept und Pegsunercept;
(iii) Calcineurin (PP-2B)-Inhibitoren/INS-Expressionsinhibitoren, wie zum Beispiel Cyclosporin A, Tacrolimus und ISA-247 von Isotechnika;
(iv) IL-1-Rezeptor-Antagonisten, wie zum Beispiel Anakinra und AMG-719 von Amgen;
(v) monoklonalen Anti-CD20-Antikörpern, wie zum Beispiel Rituximab, Ofatumumab, Ocrelizumab und TRU-015 von Trubion Pharmaceuticals;
(vi) p38-Inhibitoren, wie zum Beispiel AMG-548 (von Amgen), ARRY-797 (von Array Biopharma), Chlormethiazoledisylat, Doramapimod, PS-540446 (von BMS), SB-203580, SB-242235, SB-235699, SB-281832, SB-681323, SB-856553 (alle von GlaxoSmithKline), KC-706 (von Kemia), LEO-1606, LEO-15520 (alle von Leo), SC-80036, SD-06 (alle von Pfizer), RWJ-67657 (von R.W. Johnson), RO-3201195, RO-4402257 (alle von Roche), AVE-9940 (von Aventis), SCIO-323, SCIO-469 (alle von Scios), TA-5493 (von Tanabe Seiyaku) und VX-745 und VX-702 (alle von Vertex);
(vii) NF-kappaB (NFKB)-Aktivierungsinhibitoren, wie zum Beispiel Sulfasalazin und Iguratimod;
(viii) einem weiteren Dihydrofolatreduktase (DHFR)-Inhibitor, wie zum Beispiel Aminopterin und CH-1504 von Chelsea;
(ix) Januskinase (JAK)-Inhibitoren, wie zum Beispiel CP-690, 550 von Pfizer und INCB-18424, von Incyte;
(x) MEK-Inhibitor, wie zum Beispiel ARRY-162 von Array;
(xi) Sphingosin-1-Phosphat-Rezeptoragonisten, wie zum Beispiel fingolimod (Novartis);
(xii) Interferonen, umfassend Interferon beta 1a, wie zum Beispiel Avonex von Biogen Idec, CinnoVex von CinnaGen und Rebif von Merck Serono, und Interferon beta 1b, wie zum Beispiel Betaferon von Schering und Betaseron von Berlex;
(xiii) Immunomodulatoren, wie zum Beispiel BG-12 (Fumarsäurederivat) von Biogen Idec/Fumapharm AG; Iaquinimod (Teva und Active Biotech) oder Glatirameracetat (Teva) und
(xiv) Adenosinaminohydrolase-Inhibitoren, wie zum Beispiel Cladribin von Merck Serono.

11. Produkt, umfassend (a) Methotrexat und (b) einen DHODH-Inhibitor, wie definiert in einem der Ansprüche 1 bis 9, als kombinierte Zubereitung zur simultanen, separaten oder sequentiellen Verwendung in der Behandlung eines humanen oder Tier-Patienten, welcher leidet an oder empfänglich ist für einen(m) pathologischen Zustand oder eine(r) Krankheit, wie in Anspruch 1 definiert,
wobei
(i) das Methotrexat (a) zur Verabreichung vorgesehen ist innerhalb eines Dosierungs-Regime, das die Verabreichung von 0,015 bis 3 mg/kg/Woche Methotrexat beinhaltet und der DHODH-Inhibitor (b) für die Verabreichung in einem Dosierungs-Regime vorgesehen ist, das die Verabreichung von 0,03 bis 30 mg/kg/Tag DHODH-Inhibitor beinhaltet; oder
(ii) die Kombination vorgesehen ist zur Verwendung in der Behandlung eines humanen oder Tier-Patienten, der an Leberschädigung, Leberfibrose, Hepatitis, Zirrhose oder Leberkrebs leidet,
wobei das Produkt optional weiterhin eine aktive Verbindung (c) enthält, wie in Anspruch 10 definiert.

12. Kit aus Komponenten, umfassend (b) einen DHODH-Inhibitor, wie in einem der Ansprüche 1 bis 9 definiert, zusammen mit Anweisungen zur simultanen, separaten oder sequentiellen Verwendung in Kombination mit (a) Methotrexat zur Behandlung eines humanen oder Tier-Patienten, welcher leidet an oder empfänglich ist für einen(m) pathologischen Zustand oder Krankheit, wie in Anspruch 1 festgelegt,
wobei
(i) das Methotrexat (a) zur Verabreichung in einem Dosierungs-Regime vorgesehen ist, welches die Verabreichung von 0,015 bis 3 mg/kg/Woche Methotrexat beinhaltet und der DHODH-Inhibitor (b) zur Verabreichung in einem Dosierungs-Regime vorgesehen ist, das die Verabreichung von 0,03 bis 30 mg/kg/Tag DHODH-Inhibitor beinhaltet, oder
(ii) die Kombination zur Verwendung in der Behandlung eines humanen oder Tier-Patienten vorgesehen ist, der an Leberschädigung, Leberfibrose, Hepatitis, Zirrhose oder Leberkrebs leidet,
wobei das Kit optional ferner eine aktive Verbindung (c), wie in Anspruch 10 definiert, umfasst.

13. Packung, umfassend (b) einen DHODH-Inhibitor, wie in einem der Ansprüche 1 bis 9 definiert, und (a) Methotrexat, zur simultanen, separaten oder sequentiellen Verwendung in der Behandlung eines pathologischen Zustands oder einer Krankheit, wie in Anspruch 1 definiert,
wobei
(i) das Methotrexat (a) zur Verabreichung in einem Dosierungs-Regime vorgesehen ist, welches die Verabreichung von 0,015 bis 3 mg/kg/Woche Methotrexat beinhaltet, und der DHODH-Inhibitor (b) zur Verabreichung in einem Dosierungs-Regime vorgesehen ist, das die Verabreichung von 0,03 bis 30 mg/kg/Tag DHODH-Inhibitor beinhaltet, oder
(ii) die Kombination zur Verwendung in der Behandlung eines humanen oder Tier-Patienten vorgesehen ist, welcher an Leberschädigung, Leberfibrose, Hepatitis, Zirrhose oder Leberkrebs leidet,
wobei die Packung optional ferner eine aktive Verbindung (c), wie in Anspruch 10 definiert, umfasst.

14. DHODH-Inhibitor gemäß einem der Ansprüche 1 bis 9 zur Verwendung in der Behandlung eines humanen oder Tier-Patienten, welcher leidet an oder empfänglich ist für einen(m) pathologischen Zustand oder Krankheit gemäß Anspruch 1, wobei der humane oder Tier-Patient an Leberschädigung, Leberfibrose, Zirrhose oder Leberkrebs leidet.

15. Kombination, umfassend (a) Methotrexat und (b) einen DHODH-Inhibitor gemäß Anspruch 8 oder 9.

## Revendications

1. Combinaison de substances à utiliser simultanément, séparément ou successivement dans le traitement d'un état pathologique ou maladif susceptible d'être amélioré par l'inhibition de la dihydroorotate déshydrogénase (DHODH), choisi parmi une polyarthrite rhumatoïde, un rhumatisme psoriasique, une spondylarthrite ankylosante, une sclérose en plaques, une granulomatose de Wegener, un lupus érythémateux aigu disséminé, un psoriasis et une sarcoïdose, laquelle combinaison comprend :
a) du méthotrexate,
b) et un inhibiteur non hépato-toxique de la DHODH, de formule (I) : 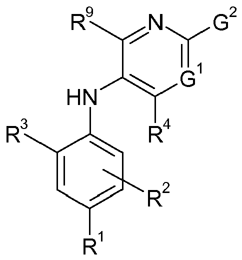 dans laquelle
- R¹ représente une entité choisie dans l'ensemble constitué par les atomes d'hydrogène et d'halogène et les groupes alkyle en C₁₋₄, cycloalkyle en C₃₋₄, trifluorométhyle et trifluorométhoxy,
- R² représente une entité choisie dans l'ensemble constitué par les atomes d'hydrogène et d'halogène et les groupes alkyle en C₁₋₄,
- R³ représente une entité choisie dans l'ensemble constitué par un groupe tétrazolyle et les groupes de formule -COOR⁵, -CONHR⁵, -SO₂NHR⁵ ou -CONHSO₂R⁵, où R⁵ représente une entité choisie dans l'ensemble constitué par un atome d'hydrogène et les groupes alkyle en C₁₋₄, à chaîne linéaire ou ramifiée,
- R⁴ représente une entité choisie dans l'ensemble constitué par un atome d'hydrogène et les groupes alkyle en C₁₋₄,
- R⁹ représente une entité choisie dans l'ensemble constitué par un atome d'hydrogène et un groupe phényle,
- G¹ représente un chaînon choisi parmi ceux symbolisés par N et par CR⁶, où R⁶ représente une entité choisie dans l'ensemble formé par les atomes d'hydrogène et d'halogène et les groupes alkyle en C₁₋₄, cycloalkyle en C₃₋₄, alcoxy en C₁₋₄, trifluorométhyle et trifluorométhoxy, les groupes hétéroaryle monocycliques azotés en C₅₋₇, les groupes hétérocyclyle monocycliques azotés en C₃₋₇, et les groupes aryle en C₆₋₁₀, lesquels groupes aryle en C₆₋₁₀, en option, portent un ou plusieurs substituant(s) choisi(s) parmi les atomes d'halogène et les groupes alkyle en C₁₋₄,
- et G² représente une entité choisie parmi les suivantes :
- un atome d'hydrogène,
- un groupe hydroxyle,
- un atome d'halogène,
- un groupe cycloalkyle en C₃₋₄,
- un groupe alcoxy en C₁₋₄,
- un groupe de formule -NR^{a}R^{b}, dans laquelle
soit R^{a} représente un groupe alkyle en C₁₋₄ et R^{b} représente une entité choisie dans l'ensemble constitué par les groupes alkyle en C₁₋₄ et (alcoxy en C₁₋₄)-alkyle en C₁₋₄,
soit R^{a} et R^{b} représentent des entités qui, conjointement avec l'atome d'azote auquel elles sont liées, forment un hétérocycle saturé de 6 à 8 chaînons qui, en option, contient un et un seul atome d'oxygène en tant qu'hétéroatome supplémentaire,
- un cycle hétéroaromatique monocyclique ou bicyclique de 5 à 10 chaînons, comportant un ou plusieurs atome(s) d'azote et portant, en option, un ou plusieurs substituant(s) choisi(s) parmi les atomes d'halogène, les groupes alkyle en C₁₋₄, alcoxy en C₁₋₄, cyclo-alkyle en C₃₋₄, cycloalcoxy en C₃₋₄, trifluorométhyle et trifluorométhoxy, et les groupes de formule -CONR⁷R⁸ dans laquelle R⁷ et R⁸ représentent des entités choisies indépendamment parmi un atome d'hydrogène et les groupes alkyle en C₁₋₄, à chaîne linéaire ou ramifiée, et cycloalkyle en C₃₋₇, ou bien R⁷ et R⁸ représentent des entités qui, conjointement avec l'atome d'azote auquel elles sont liées, forment un groupe de formule 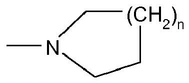 où l'indice n est un nombre entier valant de 0 à 3,
- et un groupe phényle, en option porteur d'un ou de plusieurs substituant(s) choisi(s) parmi les atomes d'halogène, les groupes alkyle en C₁₋₄, hydroxyle, alcoxy en C₁₋₄, cycloalkyle en C₃₋₄, cycloalcoxy en C₃₋₄, cyano, trifluorométhyle et trifluorométhoxy, les groupes oxadiazolyle, triazolyle, pyrazolyle et imidazolyle, lesquels groupes oxadiazolyle, triazolyle, pyrazolyle et imidazolyle, en option, portent un ou des substituant(s) alkyle en C₁₋₄ ou cycloalkyle en C₃₋₇, et les groupes de formule -CONR⁷R⁸ dans laquelle R⁷ et R⁸ représentent des entités choisies indépendamment parmi un atome d'hydrogène et les groupes alkyle en C₁₋₄, à chaîne linéaire ou ramifiée, et cycloalkyle en C₃₋₇, ou bien R⁷ et R⁸ représentent des entités qui, conjointement avec l'atome d'azote auquel elles sont liées, forment un groupe de formule 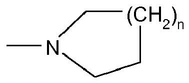 où l'indice n est un nombre entier valant de 0 à 3,
ou bien, si G¹ représente un chaînon symbolisé par CR⁶, G² représente une entité qui, conjointement avec celle représentée par R⁶, forme un groupe carbocyclique non-aromatique en C₅₋₁₀ ou un groupe aryle en C₆₋₁₀,
ou un sel ou un N-oxyde, pharmacologiquement admissible, d'un tel composé,
étant entendu
i) que le méthotrexate (a) doit être administré selon un schéma posologique impliquant l'administration de 0,015 à 3 mg/kg de méthotrexate par semaine, et l'inhibiteur de DHODH (b) doit être administré selon un schéma posologique impliquant l'administration de 0,03 à 30 mg/kg d'inhibiteur de DHODH par jour,
ii) ou que la combinaison de substances est destinée à être utilisée pour traiter un patient humain ou animal souffrant d'une insuffisance hépatique, d'une fibrose hépatique, d'une hépatite, d'une cirrhose ou d'un cancer du foie.

2. Combinaison pour utilisation, conforme à la revendication 1, dans laquelle
- R¹ représente une entité choisie dans l'ensemble constitué par les atomes d'hydrogène, de fluor, de chlore et de brome et les groupes alkyle en C₁₋₄, cycloalkyle en C₃₋₄ et trifluorométhyle,
- R² représente une entité choisie dans l'ensemble constitué par les atomes d'hydrogène et d'halogène et le groupe méthyle,
- R³ représente une entité choisie dans l'ensemble constitué par un groupe tétrazolyle et les groupes de formule -COOR⁵, -CONHR⁵, -SO₂NHR⁵ ou -CONHSO₂R⁵, où R⁵ représente une entité choisie dans l'ensemble constitué par un atome d'hydrogène et les groupes alkyle en C₁₋₄, à chaîne linéaire ou ramifiée,
- R⁴ représente une entité choisie dans l'ensemble constitué par un atome d'hydrogène et les groupes alkyle en C₁₋₄,
- R⁹ représente une entité choisie dans l'ensemble constitué par un atome d'hydrogène et un groupe phényle,
- G¹ représente un chaînon choisi parmi un atome d'azote et les chaînons symbolisés par CCl, CF, CH, C(CH₃), C(cyclopropyle), C(phényle) et C(CF₃),
- et/ou G² représente une entité choisie parmi les suivantes :
- un atome d'hydrogène,
- un atome d'halogène,
- un groupe cycloalkyle en C₃₋₄,
- un groupe alcoxy en C₁₋₂,
- un groupe de formule -NR^{a}R^{b}, dans laquelle
soit R^{a} représente un groupe alkyle en C₁₋₂ et R^{b} représente une entité choisie dans l'ensemble constitué par les groupes alkyle en C₁₋₂ et (alcoxy en C₁₋₂)-alkyle en C₁₋₂,
soit R^{a} et R^{b} représentent des entités qui, conjointement avec l'atome d'azote auquel elles sont liées, forment un hétérocycle saturé de 6 ou 7 chaînons qui, en option, contient un et un seul atome d'oxygène en tant qu'hétéroatome supplémentaire,
- un cycle hétéroaromatique monocyclique ou bicyclique de 5 à 10 chaînons, comportant un ou deux atome(s) d'azote et portant, en option, un ou plusieurs substituant(s) choisi(s) parmi les atomes d'halogène et les groupes alkyle en C₁₋₄,
- et un groupe phényle, en option porteur d'un, de deux ou de trois substituant(s) choisi(s) parmi les atomes d'halogène, les groupes alkyle en C₁₋₄, hydroxyle, alcoxy en C₁₋₄, cycloalkyle en C₃₋₄, cycloalcoxy en C₃₋₄, cyano, trifluorométhyle et trifluorométhoxy, les groupes oxadiazolyle, lesquels groupes oxadiazolyle, en option, portent un ou des substituant(s) alkyle en C₁₋₄ ou cycloalkyle en C₃₋₇, et les groupes de formule -CONR⁷R⁸ dans laquelle R⁷ et R⁸ représentent des entités choisies indépendamment parmi un atome d'hydrogène et les groupes alkyle en C₁₋₄, à chaîne linéaire ou ramifiée, et cycloalkyle en C₃₋₄, ou bien R⁷ et R⁸ représentent des entités qui, conjointement avec l'atome d'azote auquel elles sont liées, forment un groupe de formule 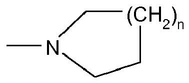 où l'indice n est un nombre entier valant 1 ou 2,
ou G¹ et G² représentent des entités qui forment conjointement un groupe carbocyclique non-aromatique en C₆ ou un groupe phényle,
ou un sel ou un N-oxyde, pharmacologiquement admissible, d'un tel composé.

3. Combinaison pour utilisation, conforme à la revendication 1 ou 2, dans laquelle
i) G² représente une entité choisie parmi les suivantes :
- un atome d'hydrogène,
- un atome de fluor,
- un groupe cyclopropyle,
- un groupe méthoxy,
- un groupe de formule -NMeEt, -NEt₂ ou -N(Me)-(CH₂)₂-OCH₃,
- un groupe morpholin-6-yle, azépan-1-yle ou pipéridin-1-yle,
- un groupe pyridinyle, pyrimidinyle, quinoléinyle ou pyrazinyle, portant, en option, un ou deux substituant(s) choisi(s) parmi un groupe méthyle et un atome de fluor,
- et un groupe phényle, en option porteur d'un, de deux ou de trois substituant(s) choisi(s) parmi les atomes de fluor et de chlore, les groupes méthyle, hydroxyle, méthoxy, éthoxy, isopropoxy, cyclopropyle, cyclopropyloxy, cyano, trifluorométhyle et trifluorométhoxy, les groupes oxadiazolyle, lesquels groupes oxadiazolyle, en option, portent un ou des substituant(s) méthyle, et les groupes de formule -CONR⁷R⁸ dans laquelle R⁷ et R⁸ représentent des entités choisies indépendamment parmi un atome d'hydrogène et les groupes méthyle, isopropyle et cyclopropyle, ou bien R⁷ et R⁸ représentent des entités qui, conjointement avec l'atome d'azote auquel elles sont liées, forment un groupe de formule 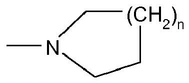 où l'indice n vaut 1,
ou G¹ et G² représentent des entités qui forment conjointement un groupe carbocyclique non-aromatique en C₆ ou un groupe phényle,
ii) et/ou G² représente un groupe choisi parmi le groupe méthoxy, le groupe cyclopropyle, et les groupes phényle, pyridinyle, quinoléinyle, pyrimidinyle et pyrazinyle, en option porteurs de substituant(s).

4. Combinaison pour utilisation, conforme à l'une des revendications 1 à 3, dans laquelle
i) R¹ représente une entité choisie dans l'ensemble constitué par les groupes alkyle en C₁₋₄, cycloalkyle en C₃₋₄ et trifluorométhyle, typiquement un groupe choisi parmi les groupes méthyle et cyclopropyle, et de préférence, un groupe cyclopropyle,
ii) et/ou R² représente une entité choisie parmi les atomes d'hydrogène et d'halogène, et typiquement, un atome d'hydrogène,
iii) et/ou R³ représente une entité choisie dans l'ensemble constitué par un groupe tétrazolyle et les groupes de formule -COOR⁵ ou -CONHR⁵, et typiquement, un groupe de formule -COOH,
iv) et/ou R⁴ représente un atome d'hydrogène ou un groupe méthyle, et typiquement, un atome d'hydrogène,
v) et/ou R⁹ représente un atome d'hydrogène,
vi) et/ou G¹ représente un chaînon choisi dans l'ensemble formé par un atome d'azote et les chaînons symbolisés par CH, C(CH₃), C(cyclopropyle), C(phényle) et C(CF₃),
vii) et/ou G² représente un groupe choisi parmi les groupes phényle, pyridinyle, quinoléinyle, pyrimidinyle et pyrazinyle, en option porteurs de substituant(s), et typiquement, un groupe choisi dans l'ensemble formé par les groupes phényle, pyridin-4-yle, quinoléin-5-yle et pyrazin-2-yle, en option porteurs de substituant(s).

5. Combinaison pour utilisation, conforme à la revendication 1 ou 2, dans laquelle
- R¹ représente une entité choisie parmi les groupes méthyle et cyclopropyle,
- R² représente un atome d'hydrogène,
- R³ représente un groupe de formule -COOH,
- R⁴ représente un atome d'hydrogène ou un groupe méthyle,
- G¹ représente un chaînon choisi parmi un atome d'azote et les chaînons symbolisés par CH, C(CH₃), C(cyclopropyle), C(phényle) et C(CF₃),
- et G² représente un groupe choisi dans l'ensemble constitué par les groupes phényle, pyridin-4-yle, quinoléin-5-yle et pyrazin-2-yle, en option porteurs de substituant(s),
et dans laquelle, en option, R⁹ représente un atome d'hydrogène.

6. Combinaison pour utilisation, conforme à la revendication 1 ou 2, dans laquelle
- R¹ représente une entité choisie parmi les groupes méthyle et cyclopropyle,
- R² représente un atome d'hydrogène,
- R³ représente un groupe de formule -COOH,
- R⁴ représente un atome d'hydrogène,
- G¹ représente un chaînon choisi parmi un atome d'azote et les chaînons symbolisés par CH, C(CH₃) et C(CF₃),
- et G² représente un groupe phényle, en option porteur d'un ou de deux substituant(s) choisi(s) parmi les atomes de fluor et de chlore, les groupes méthoxy, éthoxy, isopropoxy et trifluorométhoxy, et les groupes de formule -CONR⁷R⁸ dans laquelle R⁷ représente un atome d'hydrogène et R⁸ représente un groupe cyclopropyle, ou R⁷ et R⁸ représentent des entités qui, conjointement avec l'atome d'azote auquel elles sont liées, forment un groupe de formule 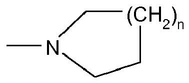
où l'indice n vaut 1.

7. Combinaison pour utilisation, conforme à la revendication 1, dans laquelle l'inhibiteur de DHODH est l'un des composés suivants :
- acide 5-cyclopropyl-2-[(2-phényl-pyrimidin-5-yl)-amino]-benzoïque
- acide 2-[(6-cyclopropyl-5-méthyl-pyridin-3-yl)-amino]-5-méthyl-benzoïque
- 5-[(2-carboxy-4-cyclopropyl-phényl)-amino]-3-méthyl-2-phényl-pyridine-1-oxyde
- acide 5-méthyl-2-({6-[3-(trifluoro-méthyl)-phényl]-pyridin-3-yl}-amino)-benzoïque
- acide 5-cyclopropyl-2-{[2-(2,6-difluoro-4-hydroxy-phényl)-pyrimidin-5-yl]-amino}-benzoïque
- acide 5-cyclopropyl-2-[(6-méthoxy-5-phényl-pyridin-3-yl)-amino]-benzoïque
- acide 2-[(5-fluoro-6-phényl-pyridin-3-yl)-amino]-5-méthyl-benzoïque
- acide 2-({6-[éthyl(méthyl)amino]-5-méthyl-pyridin-3-yl}-amino)-5-méthyl-benzoïque
- acide 5-cyclopropyl-2-[(3'-fluoro-2,4'-bipyridin-5-yl)-amino]-benzoïque
- acide 2-{[6-(diéthyl-amino)-5-méthyl-pyridin-3-yl]-amino}-5-méthyl-benzoïque
- acide 2-({6-[(2-méthoxy-éthyl)(méthyl)amino]-5-méthyl-pyridin-3-yl}-amino)-5-méthyl-benzoïque
- acide 2-[(5-chloro-6-phényl-pyridin-3-yl)-amino]-5-méthyl-benzoïque
- acide 5-cyclopropyl-2-{[2-(2-cyclopropyl-phényl)-pyrimidin-5-yl]-amino}-benzoïque
- acide 5-cyclopropyl-2-[(5-phényl-pyridin-3-yl)-amino]-benzoïque
- acide 5-méthyl-2-[(quinoléin-3-yl)-amino]-benzoïque
- acide 5-méthyl-2-[(5,6,7,8-tétrahydro-quinoléin-3-yl)-amino]-benzoïque
- acide 2-[(5-chloro-2-phényl-pyridin-3-yl)-amino]-5-méthyl-benzoïque
- acide 5-cyclopropyl-2-[(5,6-diphényl-pyridin-3-yl)-amino]-benzoïque
- acide 5-cyclopropyl-2-{[2-(2,6-difluoro-phényl)-pyrimidin-5-yl]-amino}-benzoïque
- acide 5-cyclopropyl-2-[(5-méthyl-pyridin-3-yl)-amino]-benzoïque
- acide 2-{[2-(3-cyclopropoxy-phényl)-pyrimidin-5-yl]-amino}-5-cyclopropyl-benzoïque
- acide 5-cyclopropyl-2-[(6-cyclopropyl-5-phényl-pyridin-3-yl)-amino]-benzoïque
- acide 2-{[6-(2-cyclopropyl-phényl)-5-méthyl-pyridin-3-yl]-amino}-5-méthyl-benzoïque
- acide 2-{[6-(2-cyano-phényl)-5-méthyl-pyridin-3-yl]-amino}-5-méthyl-benzoïque
- acide 2-{[2-(3-chloro-phényl)-pyrimidin-5-yl]-amino}-5-cyclopropyl-benzoïque
- acide 5-méthyl-2-{[6-phényl-5-(trifluoro-méthyl)-pyridin-3-yl]-amino}-benzoïque
- acide 2-{[6-(3-méthoxy-phényl)-5-phényl-pyridin-3-yl]-amino}-5-méthyl-benzoïque
- acide 2-[(2,3'-bipyridin-5-yl)-amino]-5-cyclopropyl-benzoïque
- acide 2-[(3'-chloro-2,4'-bipyridin-5-yl)-amino]-5-méthyl-benzoïque
- acide 5-méthyl-2-[(3-méthyl-2,2'-bipyridin-5-yl)-amino]-benzoïque
- acide 2-[(5,6-difluoro-pyridin-3-yl)-amino]-5-méthyl-benzoïque
- acide 2- {[6-(3-méthoxy-phényl)-pyridin-3-yl]-amino}-benzoïque
- acide 2- {[6-(3-éthoxy-phényl)-pyridin-3-yl]-amino}-benzoïque
- acide 2-{[6-(3-éthoxy-phényl)-pyridin-3-yl]-amino}-5-fluoro-benzoïque
- acide 2-{[6-(3-éthoxy-phényl)-5-méthyl-pyridin-3-yl]-amino}-benzoïque
- acide 2-{[6-(3-éthoxy-phényl)-pyridin-3-yl]-amino}-5-méthyl-benzoïque
- acide 2-{[6-(3-éthoxy-phényl)-5-méthyl-pyridin-3-yl]-amino}-5-méthyl-benzoïque
- acide 2-{[6-(3-éthoxy-2-fluoro-phényl)-pyridin-3-yl]-amino}-benzoïque
- acide 2-{[6-(3-éthoxy-phényl)-4-méthyl-pyridin-3-yl]-amino}-5-méthyl-benzoïque
- acide 2-{[6-(3-éthoxy-phényl)-4-méthyl-pyridin-3-yl]-amino}-benzoïque
- acide 5-bromo-2-{[6-(3-éthoxy-phényl)-pyridin-3-yl]-amino}-benzoïque
- acide 5-chloro-2-{[6-(3-éthoxy-phényl)-pyridin-3-yl]-amino}-benzoïque
- acide 2-{[6-(5-éthoxy-2-fluoro-phényl)-pyridin-3-yl]-amino}-benzoïque
- acide 2-{[6-(3-éthoxy-phényl)-5-méthyl-pyridin-3-yl]-amino}-5-(trifluoro-méthyl)-benzoïque
- acide 2-{[6-(3-méthoxy-phényl)-5-méthyl-pyridin-3-yl]-amino}-5-(trifluoro-méthyl)-benzoïque
- acide 2-{[6-(3-méthoxy-phényl)-5-méthyl-pyridin-3-yl]-amino}-5-méthyl-benzoïque
- acide 2-{[6-(3-méthoxy-phényl)-5-méthyl-pyridin-3-yl]-amino}-6-méthyl-benzoïque
- acide 5-fluoro-2-{[6-(3-méthoxy-phényl)-5-méthyl-pyridin-3-yl]-amino}-benzoïque
- acide 2-{[6-(5-éthoxy-2-fluoro-phényl)-pyridin-3-yl]-amino}-5-méthyl-benzoïque
- acide 2-{[6-(2-fluoro-5-méthoxy-phényl)-5-méthyl-pyridin-3-yl]-amino}-5-méthyl-benzoïque
- 2-{[6-(2-fluoro-5-méthoxy-phényl)-5-méthyl-pyridin-3-yl]-amino}-5-méthyl-benzoate d'éthyle
- acide 2-{[6-(2-fluoro-phényl)-pyridin-3-yl]-amino}-5-méthyl-benzoïque
- acide 2-{[6-(3-méthoxy-phényl)-5-phényl-pyridin-3-yl]-amino}-5-méthyl-benzoïque
- 2-{[6-(3-méthoxy-phényl)-5-phényl-pyridin-3-yl]-amino}-5-méthyl-benzoate d'éthyle
- acide 5-méthyl-2-[(5-méthyl-6-phényl-pyridin-3-yl)-amino]-benzoïque
- 5-méthyl-2-[(5-méthyl-6-phényl-pyridin-3-yl)-amino]-benzoate d'éthyle
- acide 5-méthyl-2-({5-méthyl-6-[3-(trifluoro-méthoxy)-phényl]-pyridin-3-yl}-amino)-benzoïque
- 5-méthyl-2-({5-méthyl-6-[3-(trifluoro-méthoxy)-phényl]-pyridin-3-yl}-amino)-benzoate d'éthyle
- acide 2-{[5-cyclopropyl-6-(3-méthoxy-phényl)-pyridin-3-yl]-amino}-5-méthyl-benzoïque
- 2-{[5-cyclopropyl-6-(3-méthoxy-phényl)-pyridin-3-yl]-amino}-5-méthyl-benzoate d'éthyle
- acide 2-{[6-(2-fluoro-5-isopropoxy-phényl)-pyridin-3-yl]-amino}-5-méthyl-benzoïque
- acide 2-{[6-(3-isopropoxy-phényl)-5-méthyl-pyridin-3-yl]-amino}-5-méthyl-benzoïque
- 2-{[6-(3-isopropoxy-phényl)-5-méthyl-pyridin-3-yl]-amino}-5-méthyl-benzoate d'éthyle
- acide 2-{[6-(3-cyclopropoxy-phényl)-5-méthyl-pyridin-3-yl]-amino}-5-méthyl-benzoïque
- 2-{[6-(3-cyclopropoxy-phényl)-5-méthyl-pyridin-3-yl]-amino}-5-méthyl-benzoate de tertiobutyle
- acide 2-{[6-(2-chloro-phényl)-5-méthyl-pyridin-3-yl]-amino}-5-méthyl-benzoïque
- 2-{[6-(2-chloro-phényl)-5-méthyl-pyridin-3-yl]-amino}-5-méthyl-benzoate de tertiobutyle
- acide 2-{[6-(3-carbamyl-phényl)-5-méthyl-pyridin-3-yl]-amino}-5-méthyl-benzoïque
- 2-{[6-(3-carbamyl-phényl)-5-méthyl-pyridin-3-yl]-amino}-5-méthyl-benzoate d'éthyle
- acide 2-{[6-(2-fluoro-5-méthoxy-phényl)-4-méthyl-pyridin-3-yl]-amino}-5-méthyl-benzoïque
- 2-{[6-(2-fluoro-5-méthoxy-phényl)-4-méthyl-pyridin-3-yl]-amino}-5-méthyl-benzoate d'éthyle
- acide 2-{[6-(3-méthoxy-phényl)-5-(trifluoro-méthyl)-pyridin-3-yl]-amino}-5-méthyl-benzoïque
- 2-{[6-(3-méthoxy-phényl)-5-(trifluoro-méthyl)-pyridin-3-yl]-amino}-5-méthyl-benzoate d'éthyle
- acide 2-({6-[3-(diméthyl-carbamyl)-phényl]-5-méthyl-pyridin-3-yl}-amino)-5-méthyl-benzoïque
- 2-({6-[3-(diméthyl-carbamyl)-phényl]-5-méthyl-pyridin-3-yl}-amino)-5-méthyl-benzoate d'éthyle
- acide 2-{[6-(3-isopropoxy-phényl)-5-méthyl-pyridin-3-yl]-amino}-3-méthyl-benzoïque
- 2-{[6-(3-isopropoxy-phényl)-5-méthyl-pyridin-3-yl]-amino}-3-méthyl-benzoate de tertiobutyle
- acide 3-méthyl-2-[(5-méthyl-6-phényl-pyridin-3-yl)-amino]-benzoïque
- 3-méthyl-2-[(5-méthyl-6-phényl-pyridin-3-yl)-amino]-benzoate de tertiobutyle
- acide 2-{[6-(2-chloro-phényl)-pyridin-3-yl]-amino}-5-méthyl-benzoïque
- 2-{[6-(2-chloro-phényl)-pyridin-3-yl]-amino}-5-méthyl-benzoate de tertiobutyle
- acide 3-fluoro-2-{[6-(3-méthoxy-phényl)-5-méthyl-pyridin-3-yl]-amino}-benzoïque
- 3-fluoro-2-{[6-(3-méthoxy-phényl)-5-méthyl-pyridin-3-yl]-amino}-benzoate de tertiobutyle
- acide 5-cyclopropyl-2-({5-méthyl-6-[3-(trifluoro-méthoxy)-phényl]-pyridin-3-yl}-amino)-benzoïque
- 5-cyclopropyl-2-({5-méthyl-6-[3-(trifluoro-méthoxy)-phényl]-pyridin-3-yl}-amino)-benzoate d'éthyle
- acide 5-cyclopropyl-2-[(5-méthyl-6-phényl-pyridin-3-yl)-amino]-benzoïque
- 5-cyclopropyl-2-[(5-méthyl-6-phényl-pyridin-3-yl)-amino]-benzoate d'éthyle
- acide 5-méthyl-2-({5-méthyl-6-[2-(trifluoro-méthyl)-phényl]-pyridin-3-yl}-amino)-benzoïque
- 5-méthyl-2-({5-méthyl-6-[2-(trifluoro-méthyl)-phényl]-pyridin-3-yl}-amino)-benzoate d'éthyle
- acide 2-{[6-(3-chloro-phényl)-5-méthyl-pyridin-3-yl]-amino}-5-méthyl-benzoïque
- 2-{[6-(3-chloro-phényl)-5-méthyl-pyridin-3-yl]-amino}-5-méthyl-benzoate de tertiobutyle
- acide 2-{[6-(2-fluoro-phényl)-5-méthyl-pyridin-3-yl]-amino}-5-méthyl-benzoïque
- 2-{[6-(2-fluoro-phényl)-5-méthyl-pyridin-3-yl]-amino}-5-méthyl-benzoate de tertiobutyle
- acide 5-méthyl-2-{[5-méthyl-6-(quinolém-5-yl)-pyridin-3-yl]-amino}-benzoïque
- 5-méthyl-2-{[5-méthyl-6-(quinoléin-5-yl)-pyridin-3-yl]-amino}-benzoate de tertiobutyle
- acide 2-[(3'-fluoro-3-méthyl-2,4'-bipyridin-5-yl)-amino]-5-méthyl-benzoïque
- 2-[(3'-fluoro-3-méthyl-2,4'-bipyridin-5-yl)-amino]-5-méthyl-benzoate de tertiobutyle
- acide 5-méthyl-2-{[5-méthyl-6-(pyrazin-2-yl)-pyridin-3-yl]-amino}-benzoïque
- 5-méthyl-2-{[5-méthyl-6-(pyrazin-2-yl)-pyridin-3-yl]-amino}-benzoate de tertiobutyle
- acide 5-cyclopropyl-2-{[6-phényl-5-(trifluoro-méthyl)-pyridin-3-yl]-amino}-benzoïque
- 5-cyclopropyl-2-{[6-phényl-5-(trifluoro-méthyl)-pyridin-3-yl]-amino}-benzoate d'éthyle
- acide 5-cyclopropyl-2-{[6-(3-méthoxy-phényl)-5-(trifluoro-méthyl)-pyridin-3-yl]-amino}-benzoïque
- 5-cyclopropyl-2-{[6-(3-méthoxy-phényl)-5-(trifluoro-méthyl)-pyridin-3-yl]-amino}-benzoate d'éthyle
- acide 5-chloro-2-{[6-(2-fluoro-phényl)-pyridin-3-yl]-amino}-benzoïque
- acide 5-chloro-2-{[6-(2-chloro-phényl)-pyridin-3-yl]-amino}-benzoïque
- acide 5-chloro-2-{[6-(quinoléin-5-yl)-pyridin-3-yl]-amino}-benzoïque
- acide 2-{[6-(2-chloro-phényl)-pyridin-3-yl]-amino}-5-cyclopropyl-benzoïque
- 2-{[6-(2-chloro-phényl)-pyridin-3-yl]-amino}-5-cyclopropyl-benzoate d'éthyle
- acide 5-chloro-2-({6-[2-(trifluoro-méthyl)-phényl]-pyridin-3-yl}-amino)-benzoïque
- acide 5-fluoro-2-({6-[2-(trifluoro-méthyl)-phényl]-pyridin-3-yl}-amino)-benzoïque
- acide 2-[(3'-fluoro-2,4'-bipyridin-5-yl)-amino]-5-méthyl-benzoïque
- acide 2-{[2-(2-fluoro-phényl)-pyrimidin-5-yl]-amino}-5-méthyl-benzoïque
- 2-{[2-(2-fluoro-phényl)-pyrimidin-5-yl]-amino}-5-méthyl-benzoate de tertiobutyle
- acide 2-{[6-(2,6-difluoro-phényl)-pyridin-3-yl]-amino}-5-méthyl-benzoïque
- 2-{[6-(2,6-difluoro-phényl)-pyridin-3-yl]-amino}-5-méthyl-benzoate d'éthyle
- acide 2-{[2-(2-chloro-phényl)-pyrimidin-5-yl]-amino}-5-cyclo-propyl-benzoïque
- 2-{[2-(2-chloro-phényl)-pyrimidin-5-yl]-amino}-5-cyclopropyl-benzoate de méthyle
- acide 2-{[2-(2-chloro-phényl)-pyrimidin-5-yl]-amino}-5-méthyl-benzoïque
- 2-{[2-(2-chloro-phényl)-pyrimidin-5-yl]-amino}-5-méthyl-benzoate de tertiobutyle
- acide 5-méthyl-2-({5-méthyl-6-[3-(pyrrolidin-1-yl-carbonyl)-phényl]-pyridin-3-yl}-amino)-benzoïque
- acide 2-({6-[3-(cyclopropyl-carbamyl)-phényl]-5-méthyl-pyridin-3-yl}-amino)-5-méthyl-benzoïque
- acide 5-cyclopropyl-2-{[2-(2-fluoro-phényl)-pyrimidin-5-yl]-amino}-benzoïque
- acide 2-({2-[2-(trifluoro-méthyl)-phényl]-pyrimidin-5-yl}-amino)-5-cyclopropyl-benzoïque
- acide 2-{[2-(ortho-tolyl)-pyrimidin-5-yl]-amino}-5-cyclopropyl-benzoïque
- acide 2-{[2-(2-cyclopropoxy-phényl)-pyrimidin-5-yl]-amino}-5-cyclopropyl-benzoïque
- acide 2-{[2-(2,5-difluoro-phényl)-pyrimidin-5-yl]-amino}-5-cyclo-propyl-benzoïque
- acide 2-{[2-(2,3-difluoro-phényl)-pyrimidin-5-yl]-amino}-5-cyclo-propyl-benzoïque
- acide 2-{[2-(2-fluoro-5-chloro-phényl)-pyrimidin-5-yl]-amino}-5-cyclopropyl-benzoïque
- acide 2-({2-[2-(trifluoro-méthyl)-phényl]-pyrimidin-5-yl}-amino)-5-méthyl-benzoïque
- acide 2-({2-[2-fluoro-5-(trifluoro-méthoxy)-phényl]-pyrimidin-5-yl}-amino)-5-cyclopropyl-benzoïque
- acide 2-({6-[2-(trifluoro-méthyl)-phényl]-pyridin-3-yl}-amino)-5-méthyl-benzoïque
- acide 2-[(6-phényl-pyridin-3-yl)-amino]-5-cyclopropyl-benzoïque
- acide 2-{[6-(2-fluoro-phényl)-pyridin-3-yl]-ammo}-5-cyclopropyl-benzoïque
- acide 2-{[6-(3,5-difluoro-pyridin-4-yl)-pyridin-3-yl]-amino}-5-méthyl-benzoïque
- acide 2-({6-[3-(cyclopropyl-carbamyl)-phényl]-pyridin-3-yl}-amino)-5-méthyl-benzoïque
- acide 2-{[6-(2,4-difluoro-phényl)-pyridin-3-yl]-amino}-5-méthyl-benzoïque
- acide 2-{[6-(2,5-difluoro-phényl)-pyridin-3-yl]-amino}-5-méthyl-benzoïque
- acide 2-{[6-(2-fluoro-phényl)-pyridin-3-yl]-amino}-5-cyclopropyl-3-fluoro-benzoïque
- acide 2-{[6-(2,3,6-trifluoro-phényl)-pyridin-3-yl]-amino}-5-méthyl-benzoïque
- acide 2-({6-[3-(5-méthyl-l,3,4-oxadiazol-2-yl)-phényl]-pyridin-3-yl}-amino)-5-méthyl-benzoïque
- acide 2-{[5-méthyl-6-(pyrimidin-5-yl)-pyridin-3-yl]-amino}-5-méthyl-benzoïque
- acide 2-{[6-(2,3-difluoro-phényl)-pyridin-3-yl]-amino}-5-méthyl-benzoïque
- acide 2-{[6-(5-fluoro-2-méthoxy-phényl)-5-méthyl-pyridin-3-yl]-amino}-5-méthyl-benzoïque
- acide 2-{[6-(4-carbamyl-phényl)-5-méthyl-pyridin-3-yl]-amino}-5-méthyl-benzoïque
ou un sel ou un N-oxyde, pharmacologiquement admissible, de l'un de ces composés.

8. Combinaison pour utilisation, conforme à la revendication 1, dans laquelle l'inhibiteur de DHODH est l'un des composés suivants :
- acide 5-méthyl-2-({6-[3-(trifluoro-méthyl)-phényl]-pyridin-3-yl}-amino)-benzoïque
- acide 5-cyclopropyl-2-{[2-(2,6-difluoro-phényl)-pyrimidin-5-yl]-amino}-benzoïque
- acide 2-{[6-(2,6-difluoro-phényl)-pyridin-3-yl]-amino}-5-méthyl-benzoïque
- acide 2-({6-[3-(cyclopropyl-carbamyl)-phényl]-5-méthyl-pyridin-3-yl}-amino)-5-méthyl-benzoïque
ou un sel ou un N-oxyde, pharmacologiquement admissible, de l'un de ces composés.

9. Combinaison pour utilisation, conforme à la revendication 8, dans laquelle l'inhibiteur de DHODH est de l'acide 5-cyclopropyl-2-{[2-(2,6-difluoro-phényl)-pyrimidin-5-yl]-amino}-benzoïque ou un sel ou un N-oxyde, pharmacologiquement admissible, de ce composé.

10. Combinaison pour utilisation, conforme à l'une des revendications précédentes,
1) dans laquelle les ingrédients actifs (a) et (b) font partie d'une et d'une seule composition pharmaceutique,
2) et/ou laquelle combinaison comprend en outre un autre composé (c), choisi parmi les suivants :
i) les anticorps monoclonaux anti-TNFα, comme les Infliximab, Certolizumab pegol, Golimumab et Adalimumab, et l'AME-527 d'Applied Molecular Evolution ;
ii) les antagonistes de TNFα, comme les Etanercept, Lenercept, Onercept et Pegsunercept ;
iii) les inhibiteurs de calcineurine (PP2B) / inhibiteurs de l'expression d'INS, comme la cyclosporine A, le Tacrolimus et l'ISA-247 d'Isotechnika ;
iv) les antagonistes des récepteurs d'IL-1, comme l'Anakinra et l'AMG-719 d'Amgen ;
v) les anticorps monoclonaux anti-CD20, comme les Rituximab, Ofatumumab et Ocrelizumab, et le TRU-015 de Trubion Pharmaceuticals ;
vi) les inhibiteurs de p38, comme l'AMG-548 d'Amgen, l'ARRY-797 d'Array Biopharma, le Chlorméthiazole édisylate, le Doramapimod, le PS-540446 de BMS, les SB-203580, SB-242235, SB-235699, SB-281832, SB-681323 et SB-856553 de GlaxoSmith-Kline, le KC-706 de Kemia, les LEO-1606 et LEO-15520 de Leo, les SC-80036 et SD-06 de Pfizer, le RWJ-67657 de R.W. Johnson, les RO-3201195 et RO-4402257 de Roche, l'AVE-9940 d'Aventis, les SCIO-323 et SCIO-469 de Scios, le TA-5493 de Tanabe Seiyaku, et les VX-745 et VX-702 de Vertex ;
vii) les inhibiteurs de l'activation de NF-κB (NFKB), comme la Sulfasalazine et l'Iguratimod ;
viii) un autre inhibiteur de la DHFR (dihydrofolate réductase), comme l'Aminoptérine et le CH-1504 de Chelsea ;
ix) les inhibiteurs de JAK (Janus kinase), comme les CP-690 et 550 de Pfizer et l'INCB-18424 d'Incyte,
x) les inhibiteurs de MEK, comme l'ARRY-162 d'Array ;
xi) les agonistes des récepteurs de la sphingosine-1-phosphate, comme le fingolimod de Novartis ;
xii) les interférons, y compris l'interféron β-1a, comme l'Avonex de Biogen Idec, le CinnoVex de CinnaGen et le Rebif de Merck Serono, et l'interféron β-1b, comme le Betaferon de Schering et le Betaseron de Berlex ;
xiii) les immunomodulateurs, comme le BG-12, dérivé de l'acide fumarique, de Biogen Idec et Fumapharm AG, le laquinimod de Teva et Active Biotech, et l'acétate de glatiramère de Teva ;
xiv) et les inhibiteurs de l'adénosine aminohydrolase, comme la cladribine de Merck Serono.

11. Produit comprenant :
a) du méthotrexate
b) et un inhibiteur de la DHODH (dihydroorotate déshydrogénase), tel que défini dans l'une des revendications 1 à 9,
sous forme d'une préparation combinée de substances à utiliser simultanément, séparément ou successivement dans le traitement d'un patient humain ou animal souffrant d'un état pathologique ou maladif tel que défini dans la revendication 1 ou prédisposé à un tel état,
étant entendu
i) que le méthotrexate (a) doit être administré selon un schéma posologique impliquant l'administration de 0,015 à 3 mg/kg de méthotrexate par semaine, et l'inhibiteur de DHODH (b) doit être administré selon un schéma posologique impliquant l'administration de 0,03 à 30 mg/kg d'inhibiteur de DHODH par jour,
ii) ou que la combinaison de substances est destinée à être utilisée pour traiter un patient humain ou animal souffrant d'une insuffisance hépatique, d'une fibrose hépatique, d'une hépatite, d'une cirrhose ou d'un cancer du foie,
lequel produit comprend en outre, en option, un composé actif (c) tel que défini dans la revendication 10.

12. Nécessaire en plusieurs parties, comprenant :
b) un inhibiteur de la DHODH, tel que défini dans l'une des revendications 1 à 9, conjointement avec un mode d'emploi en vue de l'utiliser en combinaison, simultanément, séparément ou successivement avec
a) du méthotrexate,
pour le traitement d'un patient humain ou animal souffrant d'un état pathologique ou maladif tel que défini dans la revendication 1 ou prédisposé à un tel état,
étant entendu
i) que le méthotrexate (a) doit être administré selon un schéma posologique impliquant l'administration de 0,015 à 3 mg/kg de méthotrexate par semaine, et l'inhibiteur de DHODH (b) doit être administré selon un schéma posologique impliquant l'administration de 0,03 à 30 mg/kg d'inhibiteur de DHODH par jour,
ii) ou que la combinaison est destinée à être utilisée pour traiter un patient humain ou animal souffrant d'une insuffisance hépatique, d'une fibrose hépatique, d'une hépatite, d'une cirrhose ou d'un cancer du foie,
lequel nécessaire comprend en outre, en option, un composé actif (c) tel que défini dans la revendication 10.

13. Paquet comprenant :
b) un inhibiteur de la DHODH, tel que défini dans l'une des revendications 1 à 9,
a) et du méthotrexate,
destinés à être utilisés simultanément, séparément ou successivement pour le traitement d'un état pathologique ou maladif tel que défini dans la revendication 1,
étant entendu
i) que le méthotrexate (a) doit être administré selon un schéma posologique impliquant l'administration de 0,015 à 3 mg/kg de méthotrexate par semaine, et l'inhibiteur de DHODH (b) doit être administré selon un schéma posologique impliquant l'administration de 0,03 à 30 mg/kg d'inhibiteur de DHODH par jour,
ii) ou que la combinaison est destinée à être utilisée pour traiter un patient humain ou animal souffrant d'une insuffisance hépatique, d'une fibrose hépatique, d'une hépatite, d'une cirrhose ou d'un cancer du foie,
lequel paquet comprend en outre, en option, un composé actif (c) tel que défini dans la revendication 10.

14. Inhibiteur de la DHODH, tel que défini dans l'une des revendications 1 à 9, pour utilisation dans le traitement d'un patient humain ou animal souffrant d'un état pathologique ou maladif tel que défini dans la revendication 1 ou prédisposé à un tel état, lequel patient humain ou animal souffre d'une insuffisance hépatique, d'une fibrose hépatique, d'une cirrhose ou d'un cancer du foie.

15. Combinaison comprenant
a) du méthotrexate
b) et un inhibiteur de la DHODH, tel que défini dans la revendication 8 ou 9.
